# EUROPEAN PATENT SPECIFICATION

(11) **EP 2 955 232 B1**
(45) Date of publication and mention of the grant of the patent: **23.08.2017**
(21) Application number: 14172205.8
(22) Date of filing: 12.06.2014
(51) Int. Cl.: C12Q 1/68

(54) **Method for diagnosing adenomas and/or colorectal cancer (CRC) based on analyzing the gut microbiome**
Verfahren zur Diagnose von Adenomen und/oder Kolorektalkarzinom (CRC) auf Grundlage der Analyse des Darmmikrobioms
Procédé de diagnostic d'adénomes et/ou du cancer colorectal (CRC) basé sur l'analyse du microbiome intestinal

(43) Date of publication of application: 16.12.2015
(73) Proprietor: Bork, Peer, 69117 Heidelberg (DE)
(72) Inventor: Bork, Peer, 69117 Heidelberg (DE); Tap, Julien, 91300 Massy (FR); Zeller, Georg, 69115Heidelberg (DE); Voigt, Anita, 69121 Heidelberg (DE); Kultima, Jens Roat, SE-330 21 Reftele (SE); Costea, Paul Igor, 69115 Heidelberg (DE); Mende, Daniel, 69115 Heidelberg (DE); Sunagawa, Shinichi, 69126 Heidelberg (DE); Sobbani, Iradj, 9410 Créteil (FR)
(74) Representative: Krauss, Jan

(56) References cited:
- WO-A2-2012/170478
- US-A1- 2014 024 036
- US-A1- 2014 107 092
- WEIGUANG CHEN ET AL: "Human intestinal lumen and mucosa-associated microbiota in patients with colorectal cancer, art e39743", PLOS ONE, PUBLIC LIBRARY OF SCIENCE, US , vol. 7, no. 6 1 June 2012 (2012-06-01), pages 1-9, XP003031297, ISSN: 1932-6203, DOI: 10.1371/JOURNAL.PONE.0039743 Retrieved from the Internet: URL:http://www.plosone.org/article/info%3A doi%2F10.1371%2Fjournal.pone.0039743 [retrieved on 2012-06-28]
- TINGTING WANG ET AL: "Structural segregation of gut microbiota between colorectal cancer patients and healthy volunteers", THE ISME JOURNAL, vol. 6, no. 2, 18 August 2011 (2011-08-18) , pages 320-329, XP055152252, ISSN: 1751-7362, DOI: 10.1038/ismej.2011.109
- J. AHN ET AL: "Human Gut Microbiome and Risk for Colorectal Cancer", JNCI JOURNAL OF THE NATIONAL CANCER INSTITUTE, vol. 105, no. 24, 6 December 2013 (2013-12-06), pages 1907-1911, XP055133612, ISSN: 0027-8874, DOI: 10.1093/jnci/djt300
- SHEN X ET AL: "molecular characterization of mucosal adherent bacteria and associations with colorectal adenomas", GUT MICROBES, AUSTIN, TEX. : LANDES BIOSCIENCE, US, vol. 1, no. 3, 1 May 2010 (2010-05-01), pages 138-147, XP008132812, ISSN: 1979-0976, DOI: 10.4161/GMIC.1.3.12360

## Description

The present invention relates to a method for diagnosing colorectal adenomas and/or colorectal cancer (CRC), comprising the steps of a) obtaining a fecal sample comprising the gut microbiome from a human patient to be diagnosed, b) detecting the fecal metagenome in said sample comprising the species i) *Fusobacterium nucleatum* subsp. *vincentii, Fusobacterium nucleatum* subsp. *animalis, Porphyromonas asaccharolytica,* and *Peptostreptococcus stomatis,* and/or ii) *Enterobacteriaceae bacterium* 9_2_54FAA, *Acidaminococcus intestini, Bacteroides stercoris,* and *Ruminococcaceae bacterium* D16, and c) concluding on colorectal adenomas and/or CRC in said patient based on an increase of the abundance of said species as detected in said sample, as well as to related methods and uses.

### Background of the invention

Colorectal carcinoma (CRC) is among the three most common cancers with more than 1.2 million new cases and about 600.000 deaths per year worldwide (Jemal et al., 2011). In most cases, initial genomic alterations, e.g. in the *APClWnt* signaling pathway, cause hyper-proliferation, which can lead to the formation of (benign) adenomas, and finally invasive carcinomas upon accumulation of further driver mutations (Cancer Genome Atlas Network, 2012; Vogelstein et al., 2013).

If CRC is diagnosed early, when it is still localized (American Joint Committee on Cancer (AJCC) stages 0, I or II), the five-year survival rate is >80%, but decreases to <10% for late diagnosis of metastasized cancer (in stage IV) (O'Connell et al., 2004). Therefore population-wide screening and prevention programs are recommended in many countries. Fecal occult blood testing (FOBT Hemoccult) is currently the standard non-invasive screening test (Levin et al., 2008; Zavoral et al., 2009).

As the majority of CRC cases is thought to be sporadic rather than due to inheritance (Lichtenstein et al., 2000), environmental risk factors have been investigated for a long time. An increased risk of CRC is not only associated with old age, inflammatory bowel disease (IBD) (Bernstein et al., 2001), and life-style related factors (e.g. lack of physical activity), but also with dietary components (Boleij and Tjalsma, 2012; Cross et al., 2010; Jemal et al., 2011; World Cancer Research Fund / American Institute for Cancer Research, 2011). There is convincing evidence that red meat increases CRC risk, whereas dietary fiber reduces it (Bingham et al., 2003; Greer and O'Keefe, 2011; World Cancer Research Fund / American Institute for Cancer Research, 2011).

More recently, microbes colonizing the human body have been suspected as cancer-promoting environmental factors. While in gastric, hepatic and cervical cancers a causal role is established for a single infectious agent in each case, namely *Helicobacter pylori,* Hepatitis B Virus and Human Papilloma Viruses, respectively (de Martel et al., 2012), in CRC, a variety of bacterial species and tumor-promoting virulence mechanisms have recently been discussed. For example *Bacteroides fragilis* strains producing genotoxins (BFTs) can induce inflammation leading to DNA damage in human cells (Goodwin et al., 2011; Toprak et al., 2006; Wu et al., 2009); similarly, *E. coli* strains harboring a genomic virulence island (*pks*) can cause DNA damage and chromosomal instability *in vivo* (Arthur et al., 2012; Cuevas-Ramos et al., 2010), and very recently *Fusobacterium nucleatum* strains were reported to promote carcinogenesis upon invasion of epithelial cells (Kostic et al., 2013; Rubinstein et al., 2013). However it remains unclear, how many CRC cases can be attributed to each of these agents, how these exactly interact with the human host or the microbial community in the gut, and whether altered microbial abundances may provide a basis for an accurate CRC screening test.

Over the first several years of life, the human skin surface, oral cavity, and gut are colonized by a tremendous diversity of bacteria, archaea, fungi, and viruses. The community formed by this complement of cells is called the human microbiome; it contains almost ten times as many cells as are in the rest of our bodies and accounts for several pounds of body weight and orders of magnitude more genes than are contained in the human genome. Under normal circumstances, these microbes are commensal, helping to digest food and to maintain the immune systems. Although the human microbiome has long been known to influence human health and disease, we have only recently begun to appreciate the breadth of its involvement. This is almost entirely due to the recent ability of high-throughput sequencing to provide an efficient and cost-effective tool for investigating the members of a microbial community and how they change. Thus, dysfunctions of the human microbiota are increasingly being linked to disease ranging from inflammatory bowel disease to diabetes to antibiotic-resistant infection, and the potential of the human microbiome as an early detection biomarker and target for therapeutic intervention is a vibrant area of current research.

Obtaining a comprehensive view of the microbial ecosystem in the patient's gut - the microbiome - has become possible with high-throughput environmental shotgun sequencing techniques (Human Microbiome Project Consortium, 2012; Qin et al., 2010), and first associations of microbiota with disease have been reported, such as obesity, type II diabetes, and atherosclerosis (e.g. Karlsson et al., 2013; Le Chatelier et al., 2013; Qin et al., 2012; Turnbaugh et al., 2009). Several medium-scale studies recently characterized the microbiota of colonic tumor biopsies compared to healthy mucosa either by quantifying the 16S rRNA phylogenetic marker gene or by metatranscriptomic sequencing (Castellarin et al., 2012; Flanagan et al., 2014; Kostic et al., 2012; Marchesi et al., 2011; McCoy et al., 2013; Tahara et al., 2014; Warren et al., 2013). Even though these consistently documented an enrichment of members of the *Fusobacterium* genus, the relevance of these or other microbial agents for non-invasive CRC screening is still unclear.

Because FOBT has limited sensitivity and specificity for CRC and does not reliably detect precancerous lesions (Allison et al., 1996; Faivre et al., 2004), there is an urgent demand for more accurate screening tests to identify patients who should undergo colonoscopy, which is considered the most effective diagnostic method (Levin et al., 2008).

WO 2012/170478, US 2014-107092, and US 2014-024036 each disclose a method for detecting colorectal cancer or adenomas by detecting the levels of bacteria species in a stool sample. In particular, the three documents indicate *Fusobacterium nucleatum* as the most relevant species.

Weiguang Chem et al., "Human intestinal lumen and mucosa-associated microbiota in patients with colorectal cancer, art e39743", vol. 7, no. 6, (2012-06-01), pages 1 - 9, PLOS ONE, discloses pyrosequencing based analysis of 16S rRNA genes to determine the overall structure of microbiota in patients with colorectal cancer and healthy controls. The overall microbial structures of cancerous tissue and noncancerous tissue were similar; howerer the tumor microbiota exhibited lower diversity.

Tingting Wang et al., "Structural segregation of gut microbiota between colorectal cancer patients and healthy volunteers", The ISME J, (2011-08-18), vol. 6, no. 2, pages 320 - 329, disclose a study where fecal bacterial diversity in CRC patients (n=46) and healthy volunteers (n=56) were profiled by 454 pyrosequencing of the V3 region of the 16S ribosomal RNA gene. Bacteria of the genera Enterococcus, Escherichia/Shigella, Klebsiella, Streptococcus and Peptostreptococcus were significantly more abundant in the gut microbiota of CRC patients, and bacteria belonging to the genus Roseburia and other butyrate-producing bacteria of the family Lachnospiraceae were less abundant.

J. Ahn et al., "Human Gut Microbiome and Risk for Colorectal Cancer", JNCI J Nat Cancer Inst, (2013-12-06), vol. 105, no. 24, pages 1907 - 1911, identified lower relative abundance of Clostridia (68.6% vs 77.8%) and increased carriage of Fusobacterium (multivariable odds ratio [OR] = 4.11; 95% confidence interval [CI] = 1.62 to 10.47) and Porphyromonas (OR = 5.17; 95% CI = 1.75 to 15.25) in CRC case subjects compared with control subjects.

It is therefore an object of the present invention to provide a more accurate screening test in order to, for example, identify patients who should then undergo colonoscopy. Other objects of the present invention will become apparent to the person of skill when studying the specification of the present invention.

In a first aspect thereof, the object of the present invention is solved by providing a method diagnosing colorectal adenomas and/or colorectal cancer (CRC), comprising the steps of a) obtaining a fecal sample comprising the gut microbiome from a human patient to be diagnosed, b) detecting the fecal metagenome in said sample comprising the species i) *Fusobacterium nucleatum* subsp. *vincentii, Fusobacterium nucleatum* subsp. *animalis, Porphyromonas asaccharolytica,* and *Peptostreptococcus stomatis,* and/or ii) *Enterobacteriaceae bacterium* 9_2_54FAA, *Acidaminococcus intestini, Bacteroides stercoris,* and *Ruminococcaceae bacterium* D16, and c) concluding on colorectal adenomas and/or CRC in said patient, wherein an increase of the abundance of said species as detected in said sample compared to a sample from a non-colorectal adenoma and/or CRC patient is indicative for CRC for the species as listed in step b i), and indicative for colorectal adenomas for the species as listed in step b ii).

Thus, preferred is a method for diagnosing colorectal adenomas, comprising the steps of a) obtaining a fecal sample comprising the gut microbiome from a human patient to be diagnosed, b) detecting the fecal metagenome in said sample comprising the species *Enterobacteriaceae bacterium* 9_2_54FAA, *Acidaminococcus intestini, Bacteroides stercoris,* and *Ruminococcaceae bacterium* D16, and optionally *Bacteroides fragilis,* and c) concluding on colorectal adenomas in said patient, wherein an increase of the abundance of said species as detected in said sample compared to a sample form a non- colorectal adenoma patient is indicative for colorectal adenomas.

Also preferred is a method for diagnosing colorectal cancer (CRC), comprising the steps of a) obtaining a fecal sample comprising the gut microbiome from a human patient to be diagnosed, b) detecting the fecal metagenome in said sample comprising the species *Fusobacterium nucleatum* subsp. *vincentii, Fusobacterium nucleatum* subsp. *animalis, Porphyromonas asaccharolytica,* and *Peptostreptococcus stomatis,* and c) concluding on CRC in said patient, wherein an increase of the abundance of said species as detected in said sample compared to a sample form a non-CRC patient is indicative for CRC.

The inventive methods can also be performed on samples derived from the fecal sample, as long as the composition of the gut microbiome can be reliably determined. Thus, optimally, the samples should be obtained and maintained using procedures that avoid harsh treatments of the samples, in order to maintain the composition of the strains as analyzed to as much as possible. Factors that should be monitored are, amongst others, temperature, humidity, and contact with air (oxygen). Suitable sampling methods are known to the person of skill (e.g. as described herein), and can be identified by the person of skill without any undue burden. The inventive methods can also be performed on a sample as obtained from said patient, e.g. on a sample as taken and suitably stored for later analysis.

In the context of the present invention, the inventors taxonomically and functionally characterized the gut microbiome based on the bacterial species as described herein, using shotgun metagenomics of fecal samples from patients with colorectal carcinomas (in all stages of CRC progression), colorectal adenomas, as well as from neoplasia-free controls. The inventors established the utility of changes in the taxonomic composition of the gut microbiome for CRC screening using study populations that comprise participants from several countries. An analysis of the functions encoded in the metagenome further revealed a surprising metabolic shift towards host glycan degradation as well as an increase in pro-inflammatory processes that distinguished CRC patients from controls.

Preferred is the method according to the present invention, wherein said CRC as diagnosed is localized CRC (stages 0, I, or II) and/or advanced colorectal adenomas. Staging is according to the AJCC guidelines. Further preferred is the method according to the present invention, wherein said CRC is further distinguished from neoplasia-free (non-colorectal adenoma) in said non-CRC patient. The present invention is particularly useful for a distinction of early stages of CRC, which is very difficult to assess with common tests, such as Hemoccult FOBT.

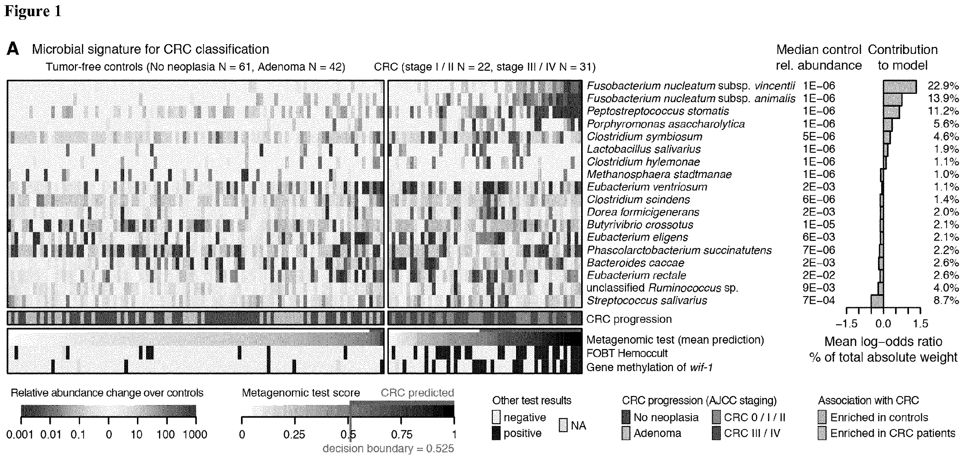

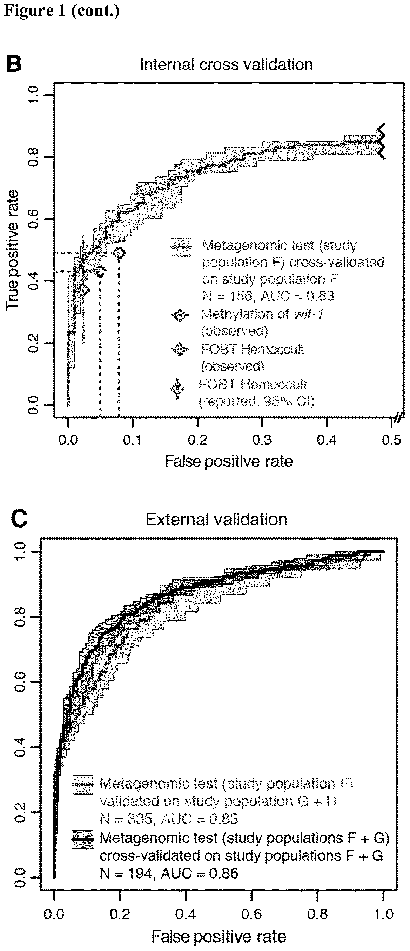

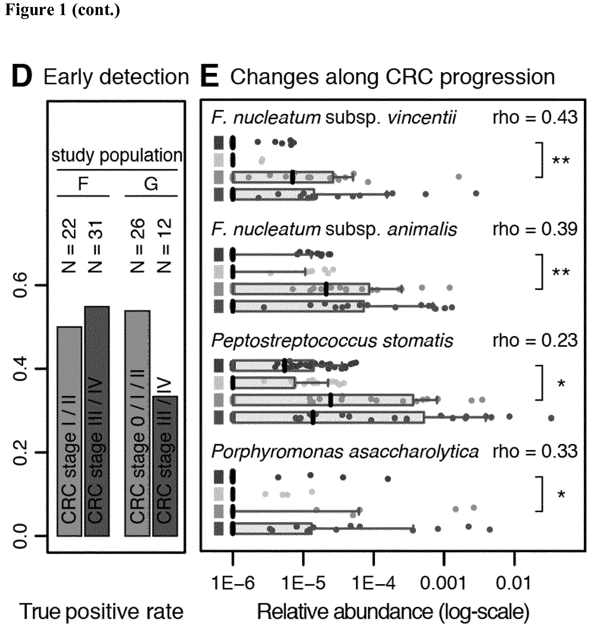

Preferred is the method according to the present invention, further comprising for CRC detecting an increase of the abundance of at least one of the species *Clostridium symbiosum, Clostridium hylemonae,* and *Lactobacillus salivarius,* and/or a decrease of the abundance of the species *Streptococcus salivarius.* These species can add even further significance to the methods of the invention. Most preferred is the detection of all 18 species as listed in Figure 1.

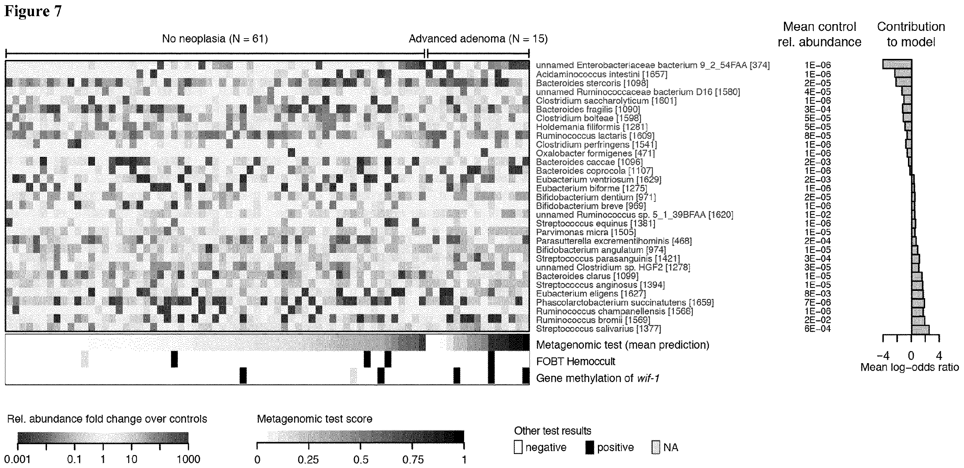

Preferred is the method according to the present invention, further comprising for colorectal adenomas detecting an increase of the abundance of at least one of the species *Clostridium saccharolyticum, Bacteroides fragilis, Clostridium bolteae, Holdermania filiformis, Ruminococcus lactaris, Clostridium perfringens, Oxalobacter formigenes, Bacteroides caccae,* and *Bacteroides coprocola* and/or a decrease of the abundance of at least one of the species *Streptococcus salivarius* and *Ruminococcus bromii.* These species can add even further significance to the methods of the invention. Most preferred is the detection of all 31 species as listed in Figure 7.

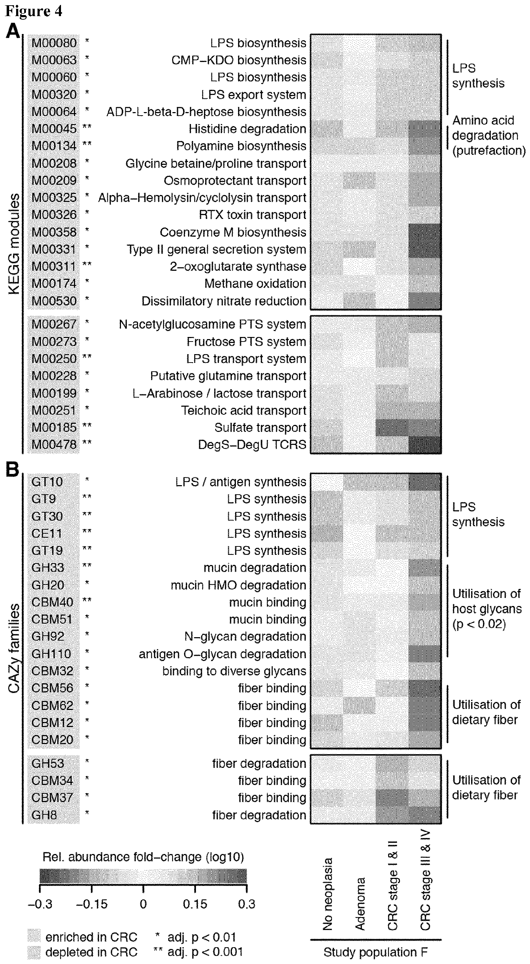

Yet another aspect of the present invention then relates to a method according to the present invention, further comprising combining said test with a method selected from detecting the level of methylation in the gene *wif-1* (see examples, below), amount (abundance) or expression of KEGG (Kyoto Encyclopedia of Genes and Genomes) module markers, amount (abundance) or expression of CAZy (Carbohydrate-Active enZYmes database) family markers, and the Hemoccult FOBT test. Preferred KEGG module markers and CAZy markers are selected from markers (e.g. genes) involved in the LPS metabolism, such as, for example, listed in Figure 4. Preferred are markers that are enriched in CRC with a p value of < 0.001.

For measuring and/or detecting of the species as described herein, standard methods can be used, such as, for example, a (preferably quantitative) method selected from the group of PCR, rtPCR, qPCR, multiplex PCR, high-throughput sequencing, metatranscriptomic sequencing, identification of strain-specific markers, such as genes and/or proteins, 16S rDNA analysis, and DNA microarray techniques. These methods also include enzymatic tests in order to identify functional changes (see, for example, Figure 4), as long as these changes for allow a species-specific analysis.

Yet another aspect of the present invention then relates to a method for monitoring the progress of colorectal adenomas and/or colorectal cancer (CRC), comprising the steps of a) obtaining a fecal sample comprising the gut microbiome from a human patient to be diagnosed, b) detecting the fecal metagenome in said sample comprising the species i) *Fusobacterium nucleatum* subsp. *vincentii, Fusobacterium nucleatum* subsp. *animalis, Porphyromonas asaccharolytica,* and *Peptostreptococcus stomatis,* and/or ii) *Enterobacteriaceae bacterium* 9_2_54FAA, *Acidaminococcus intestini, Bacteroides stercoris,* and *Ruminococcaceae bacterium* D16, and c) concluding on the progress of said colorectal adenomas and/or CRC in said patient, wherein an increase of the abundance of said species as detected in said sample compared to a sample from a non-progressing colorectal adenoma and/or CRC patient is indicative for progressive CRC for the species as listed in step b i), and indicative for progressing colorectal adenomas for the species as listed in step b ii).

Preferred is the method according to the present invention, wherein said patient having said colorectal adenomas and/or said colorectal cancer (CRC) has been diagnosed using a method according to the present invention as described above. Further preferred is the method according to the present invention, wherein said monitoring further comprises grading of said colorectal adenomas and/or said CRC into localized CRC (stages 0, I, or II) and/or advanced colorectal adenoma, also as described above.

Preferred is the method according to the present invention, further comprising combining said test with a method selected from detecting the level of methylation in the gene *wif-1,* amount, abundance or expression of KEGG module markers, amount, abundance or expression of CAZy family markers, and Hemoccult FOBT test. This should improve the test even further.

Disclosed is a method wherein said patient is undergoing treatment for colorectal adenomas and/or CRC. Said treatment can preferably be performed as described below.

In addition to the above approaches, new targets for the therapy of CRC and/or adenomas are desired. Thus, disclosed is a method for detecting and/or identifying a compound suitable for the treatment of colorectal adenomas and/or colorectal cancer (CRC), comprising the steps of a) administering a candidate compound to a mammal having a colorectal adenoma and/or CRC, and b) obtaining a fecal sample comprising the gut microbiome from said mammal, c) detecting the fecal metagenome in said sample comprising the species i) *Fusobacterium nucleatum* subsp. *vincentii, Fusobacterium nucleatum* subsp. *animalis, Porphyromonas asaccharolytica,* and *Peptostreptococcus stomatis,* and/or ii) *Enterobacteriaceae bacterium* 9_2_54FAA, *Acidaminococcus intestini, Bacteroides stercoris,* and *Ruminococcaceae bacterium* D16, and d) identifying a compound suitable for the treatment of said colorectal adenoma and/or CRC in said mammal, wherein an increase of the abundance of said species as detected in said sample compared to a sample from a non-colorectal adenoma and/or -CRC patient is indicative for a compound suitable for the treatment of CRC for the species as listed in step c i), and indicative for a compound suitable for the treatment of colorectal adenomas for the species as listed in step c ii).

The mammal and/or patient can be a rat, mouse, goat, rabbit, sheep, horse, monkey or human, preferred is a mouse, rat or human. Most preferred is a mouse.

More preferred is a method for detecting and/or identifying as disclosed, wherein said compound is selected from the group consisting of a peptide library, a combinatory library, a cell extract, in particular a plant cell extract, a "small molecular drug", an antisense oligonucleotide, an siRNA, an mRNA and an antibody or fragment thereof (such as Fab or scFv, and the like).

Disclosed is a method for detecting and/or identifying, further comprising testing said compound(s) as detected/identified for its activity on colorectal adenomas and/or CRC. Respective assays are known to the person of skill, and can be taken from the respective literature.

Disclosed is a method for detecting and/or identifying according to the present invention, wherein steps a) to d) are repeated, and, optionally, chemically modifying said compound before said repeating. The thus identified candidate compound can then, in a preferred embodiment, modified in a further step. Modification can be effected by a variety of methods known in the art, which include, without limitation, the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or isopentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group. The thus modified binding substances are than individually tested with a method of the present invention. If needed, the steps of selecting the candidate compound, modifying the compound, and testing compound can be repeated a third or any given number of times as required. The above described method is also termed "directed evolution" since it involves a multitude of steps including modification and selection, whereby binding compounds are selected in an "evolutionary" process optimizing its capabilities with respect to a particular property, e.g. its ability to act on adenomas, CRC and/or their progression.

Disclosed is a method for detecting and/or identifying according to the present invention, wherein said patient is undergoing treatment for colorectal adenomas and/or CRC. Said treatment can preferably be performed as described below.

Disclosed is a method for manufacturing a pharmaceutical composition for treating or preventing colorectal adenomas and/or CRC, comprising the steps of: performing a method for detecting and/or identifying according to the present invention, and formulating said compound as detected and identified into a pharmaceutical composition.
compound identified as outlined above, which may or may not have gone through additional rounds of modification
and selection, is admixed with suitable auxiliary substances and/or additives. Such substances comprise pharmacological acceptable substances, which increase the stability, solubility, biocompatibility, or biological half-life of the interacting compound or comprise substances or materials, which have to be included for certain routes of application like, for example, intravenous solution, sprays, band-aids or pills.

Carriers, excipients and strategies to formulate a pharmaceutical composition, for example to be administered systemically or topically, by any conventional route, in particular enterally, e.g. orally, e.g. in the form of tablets or capsules, parenterally, e.g. in the form of injectable solutions or suspensions, topically, e.g. in the form of lotions, gels, ointments or creams, or in nasal or a suppository form are well known to the person of skill and described in the respective literature.

Administration of an agent, e.g., a compound can be accomplished by any method which allows the agent to reach the target cells. These methods include, e.g., injection, deposition, implantation, suppositories, oral ingestion, inhalation, topical administration, or any other method of administration where access to the target cells by the agent is obtained. Injections can be, e.g., intravenous, intradermal, subcutaneous, intramuscular or intraperitoneal. Implantation includes inserting implantable drug delivery systems, e.g., microspheres, hydrogels, polymeric reservoirs, cholesterol matrices, polymeric systems, e.g., matrix erosion and/or diffusion systems and non-polymeric systems, e.g., compressed, fused or partially fused pellets. Suppositories include glycerin suppositories. Oral ingestion doses can be enterically coated. Inhalation includes administering the agent with an aerosol in an inhalator, either alone or attached to a carrier that can be absorbed. The agent can be suspended in liquid, e.g., in dissolved or colloidal form. The liquid can be a solvent, partial solvent or non-solvent. In many cases, water or an organic liquid can be used.

Disclosed is a pharmaceutical composition for treating or preventing adenomas and/or CRC, obtainable by a method according to the method as herein. Disclosed is a pharmaceutical composition as described herein, wherein the pharmaceutical composition further comprises additional pharmaceutically active ingredients for treating colorectal adenomas and/or CRC, such as, for example, chemotherapeutics.

Disclosed is a method for treating or preventing colorectal adenomas and/or CRC in a human patient in need thereof, comprising a diagnosis and/or monitoring according to a method according to the present invention as described herein.

Disclosed is a method comprising administering to said patient an effective amount of a compound as identified as described herein. In general, the attending physician will base a treatment on the compound as identified, and optionally also on other individual patient data (clinical data, family history, DNA, etc.), and a treatment can also be performed based on the combination of these factors. This method for example involves integrating individual diagnostic colon disease data with patient clinical information and general healthcare statistics to enable, for example, the application of personalized medicine to the patient. Significant information about drug effectiveness, drug interactions, and other patient status conditions can be used, too.

Treatment is meant to include, e.g., preventing, treating, reducing the symptoms of, or curing the disease or condition, i.e. colorectal adenomas, CRC and/or advanced adenomas. An "effective amount" is an amount of the compound(s) or the pharmaceutical composition as described herein that alleviates symptoms as found for colorectal adenomas, CRC and/or advanced colorectal adenomas, such as, for example, frequency and/or size. Alleviating is meant to include, e.g., preventing, treating, reducing the symptoms of, or curing the disease (CRC and/or advanced adenomas) or condition. Disclosed is a method for treating a subject at risk for a development and/or progression of colorectal adenomas, CRC and/or advanced colorectal adenomas, wherein a therapeutically effective amount of a compound as above is provided. Being at risk for the disease can result from, e.g., a family history of the disease, a genotype which predisposes to the disease, or phenotypic symptoms which predispose to the disease.

Disclosed is the use of a diagnostic kit comprising, in one or separate containers, materials for measuring the fecal metagenome of in a mammal, preferably a human, comprising at least one of the species *Fusobacterium nucleatum* subsp. *vincentii, Fusobacterium nucleatum* subsp. *animalis, Porphyromonas asaccharolytica, Peptostreptococcus stomatis, Enterobacteriaceae bacterium* 9_2_54FAA, *Acidaminococcus intestini, Bacteroides stercoris,* and *Ruminococcaceae bacterium* D16, for diagnosing and/or monitoring colorectal adenomas and/or CRC in a mammalian, preferably a human, patient. Preferred is the use for the species *Fusobacterium nucleatum* subsp. *vincentii, Fusobacterium nucleatum* subsp. *animalis, Porphyromonas asaccharolytica,* and *Peptostreptococcus stomatis,* and/or *Enterobacteriaceae bacterium* 9_2 54FAA, *Acidaminococcus intestini, Bacteroides stercoris,* and *Ruminococcaceae bacterium* D16.

Optionally, the kit comprises instructions for performing a method according to the present invention as described herein. The container is preferably a bottle, a vial, a syringe or test tube; and it may be a multi-use container. The container may be formed from a variety of materials such as glass or plastic.

Preferred is the use of a diagnostic kit, wherein said kit, in one or separate containers, for CRC further comprises materials for detecting an increase of the abundance of at least one of the species *Clostridium symbiosum, Clostridium hylemonae,* and *Lactobacillus salivarius,* and/or a decrease of the abundance of the species *Streptococcus salivarius.* Preferred is the use of a diagnostic kit, wherein said kit, in one or separate containers, for adenomas further comprises materials for detecting an increase increase of the abundance of at least one of the species *Clostridium saccharolyticum, Bacteroides fragilis, Clostridium bolteae, Holdermania filiformis, Ruminococcus lactaris, Clostridium perfringens, Oxalobacter formigenes, Bacteroides caccae,* and *Bacteroides coprocola* and/or a decrease of the abundance of at least one of the species *Streptococcus salivarius* and *Ruminococcus bromii.*

Preferred is the use of a diagnostic kit, wherein said kit, in one or separate containers, further comprises materials for a method such as a quantitative method, selected from the group of PCR, rtPCR, qPCR, multiplex PCR, high-throughput sequencing, metatranscriptomic sequencing, identification of strain-specific markers, such as genes and/or proteins, and 16S rDNA analysis. Also functional markers can be analyzed, as explained above.

While a combination of microbial markers with existing screening and diagnostic tools, such as FOBT and colonoscopy, appears promising for future application in mass screening, the success of any such test will depend on the development of cost-effective assays (e.g. based on PCR). The accuracy of these would have to be confirmed in large-scale comparisons to other recently introduced CRC screening tools (e.g. immunochemical FOBT (Allison et al., 2007; Hol et al., 2010) or genetic markers (Pritchard and Grady, 2011). The inventors here provide an assay showing the potential of the gut microbiome as a conceptually novel approach to CRC screening.

The present invention shall now be further described in the following examples with reference to the accompanying figures, nevertheless, without being limited thereto. In the Figures,
Figure 1 shows the signature of CRC-associated gut microbial species. (A) Relative abundances of 18 gut microbial species, collectively associated with CRC, are displayed as heatmap in the left panel as fold change over the median relative abundance observed in controls. This control median relative abundance is indicated to the right as well as their mean contribution to the classification (bars show log-odds ratios in multivariate logistic regression, numbers indicate percentage of absolute total weight). Patient subgroups in different stages of cancer progression are color-coded below the heatmap. Below, the classification score of the microbial signature (from cross validation) is shown as gray scale (see key) with the decision boundary and resulting false positives and true positives indicated in red (using colonoscopy results as a ground truth). Displayed alongside are the results of the standard Hemoccult FOBT test routinely applied for CRC screening (Allison et al., 1996) and an experimental CRC screening test based on methylation of the *wif-1* gene, a *Wnt* pathway member (Lee et al., 2009; Roperch and Sobhani). (B) Test accuracy of the metagenomic classifier is depicted as median ROC curve summarizing predictions made in ten times resampled ten-fold cross validation on study population F (grey area indicates the range between the 10th and 90th percentile of ROC curves). Additionally, the accuracy of the *wif-1* methylation test (Lee et al., 2009; Roperch and Sobhani) as well as of the FOBT test is shown. The latter is not only indicated as assessed for the same patients, but also as reported in a large-scale survey (vertical bar denotes 95% confidence interval) (Allison et al., 1996). All screening tests are evaluated relative to colonoscopy findings. (C) When applied to the larger study population G and H (335 metagenomes from several countries including 38 from German CRC patients) for external validation, the metagenomic classifier achieved very similar accuracy as in cross validation, when measured by the area under the ROC curve (AUC). When samples from study populations F and G were combined (N = 194) to retrain a classifier, its accuracy improved to 0.84 AUC (median AUC curve from ten times resampled ten-fold cross validation, area between the 10^{th} and 90^{th} percentile of ROC curves is shaded). (D) Sensitivity (TPR) of the metagenomic classifier for carcinomas in early stages (AJCC stages 0, I and II) was at least as good as for late-stage, metastasizing CRC (AJCC stages III and IV) in both study populations F and G highlighting its potential utility for early detection. (E) Although the classifier associated species with a binary grouping into cancer and non-cancer patients, several of them exhibited gradual abundance changes over the CRC progression from healthy participants over adenoma, early and late-stage cancer patients (see key below (A)); displayed are the five most discriminative CRC marker species, each of which shows a Spearman correlation (rho) with cancer progression (grouped as in (A)) that is stronger than 0.2 with p-values < 0.001. Vertical black lines indicate median relative abundance with grey boxes denoting the interquartile range; grey whiskers extend to the 5^{th} and 95^{th} percentile.

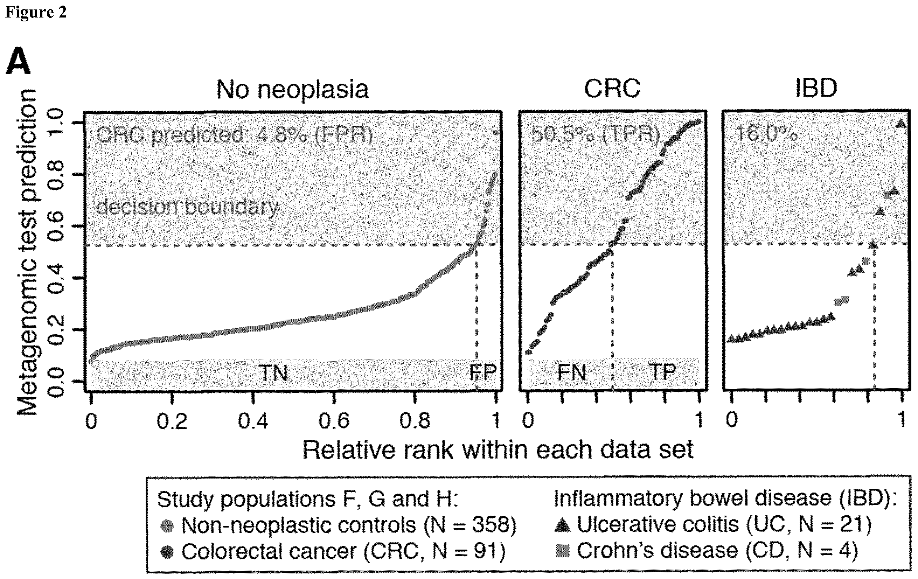

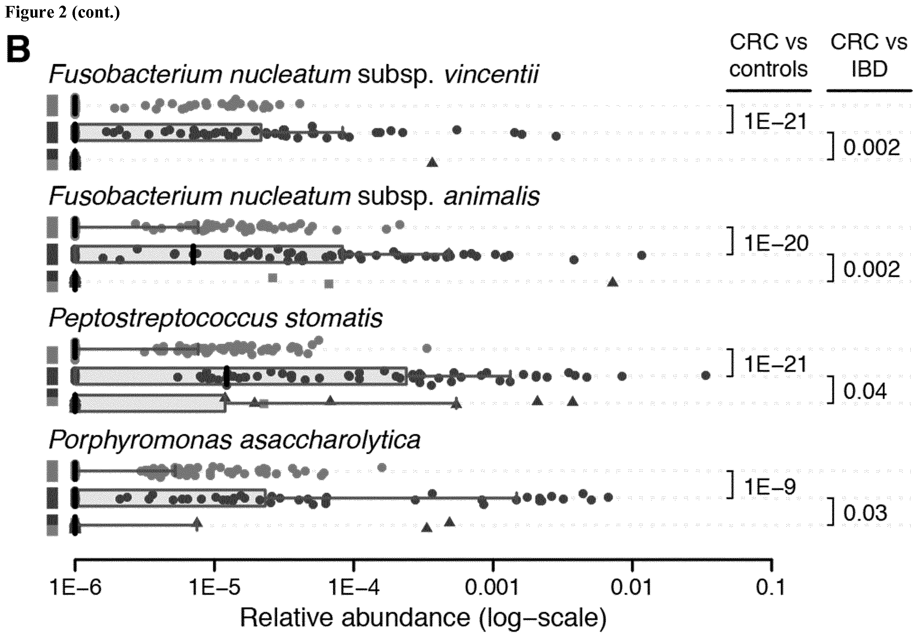

Figure 2 shows the comparison of the CRC microbial signature to IBD microbiomes. (A) Ranked predictions of the metagenomic classifier (mean test prediction for study populations F, G and H) are plotted for each individual (labels on top indicate grouping) with the percentage of positive CRC predictions annotated in red. Proportions of true negatives (TN), false positives (FP), false negatives (FN) and true positives (TP) are shown at bottom for a decision boundary of 0.6 (see Fig. 1). Application of the CRC classifier to metagenomes from Ulcerative colitis (UC) and Crohn's disease (CD) patients suggests moderately increased false positive rates for inflammatory bowel disease (IBD) patients. (B) Relative abundance distributions of the five most discriminative markers for CRC classification (see Fig. 1A) are plotted for each patient subgroup, including UC and CD (see key). All markers showed significantly stronger association with (enrichment in) CRC than with IBD (UC and CD tested together). Boxplots are as defined in Fig. 1E.

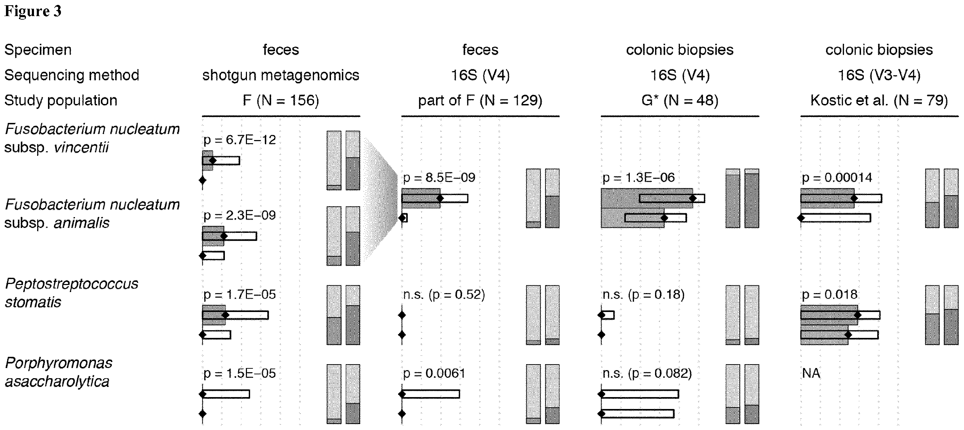

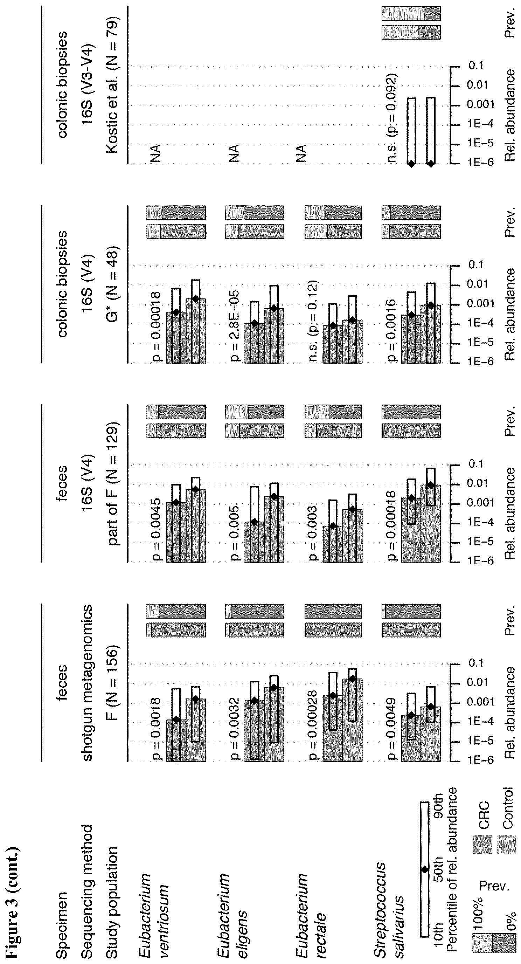

Figure 3 shows the consistency of CRC marker species abundances in fecal metagenomes and 16S rRNA profiles of tumor biopsies. Horizontal bars show CRC-associated changes in median relative abundance (rel. abundance) of the marker species in the metagenomic CRC classifier that significantly differ in abundance between CRC patients and tumor-free controls. They are compared to 16S OTU abundances from a subset of fecal samples from study population F as well as two groups of patients in which microbial communities on tumor biopsies and healthy colonic mucosa were profiled and compared. Boxes denote the interval between the 10th and 90th percentile of relative abundance. Metagenomic marker species were matched to 16S OTUs using a best-hit approach for the amplified 16S rRNA gene regions (NA, not matched), both *Fusobacterium nucleatum* sub spp. were matched to the same 16S OTU. Significance was assessed by unpaired and paired Wilcoxon tests for fecal and biopsy data sets, respectively (n.s., not significant). Note that for the majority of the species shown, the significance for distinguishing healthy and CRC patients is higher (lower p-value) in metagenomic than 16S readouts. Vertical bars display the prevalence (prev.) of these marker species in CRC and control(s) (tissue) (percentage of individuals in which these species/OTUs were detected with a relative abundance exceeding 1E-5).
Figure 4 shows the functional changes derived from the CRC-associated metagenome. (A) Significant changes in relative abundance of genes summarized by KEGG module annotations between cancer and non-cancer metagenomes are shown for cases with a >1.33 fold change and an FDR adjusted p-value < 0.01. General trends in functional potential, such as enrichment of lipopolysaccharide (LPS) metabolism, and putrefaction in the CRC microbiome are summarized to the right of the heatmap. (B) Significant relative abundance changes of genes summarized by CAZy family annotation with a >1.33 fold change and an FDR-adjusted p-value < 0.01. A metabolic switch to degradation of host carbohydrates, e.g. mucins, in CRC metagenomes, as well as a CRC-associated increase in metabolism of potentially pro-inflammatory bacterial cell wall components, such as lipopolysaccharide (LPS) is annotated to the right.

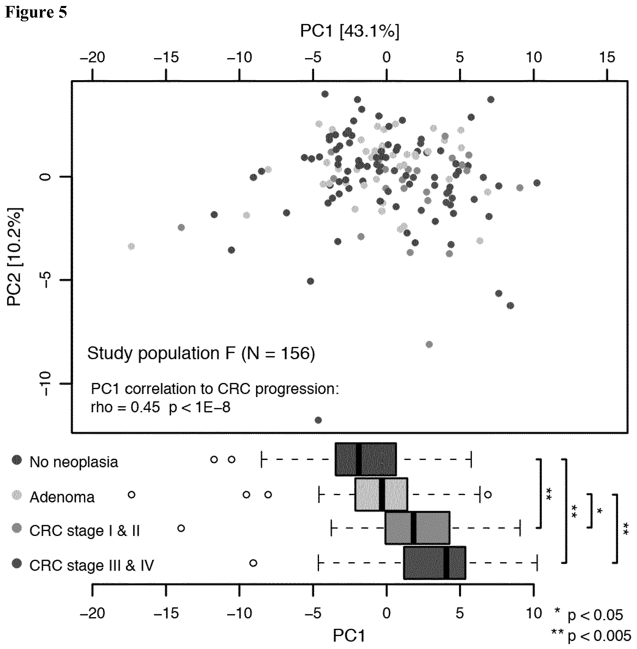

Figure 5 shows that functional changes correlate with CRC progression. Principal component analysis of (log10-transformed) relative abundances of CRC-associated functional categories (as in Fig. 4) revealed cancer progression as a dominant source of variation (each dot corresponds to a participant of study population F, color-coded by patient subgroup). The first principal component (PC1), accounting for 43% of the variation in CRC-associated functional changes, correlated with cancer progression (Spearman correlation of 0.45, p < 1E-8) and stratified non-neoplastic, adenoma early and late CRC patients (color-coded). Shown below are boxplots of each patient's PC1 value grouped by cancer progression. Significant differences between groups were established using pairwise Wilcoxon tests (see bottom right legend). Boxes denote interquartile ranges (IQR) with the median as a black line and whiskers extending up to the most extreme points within 1.5-fold IQR.

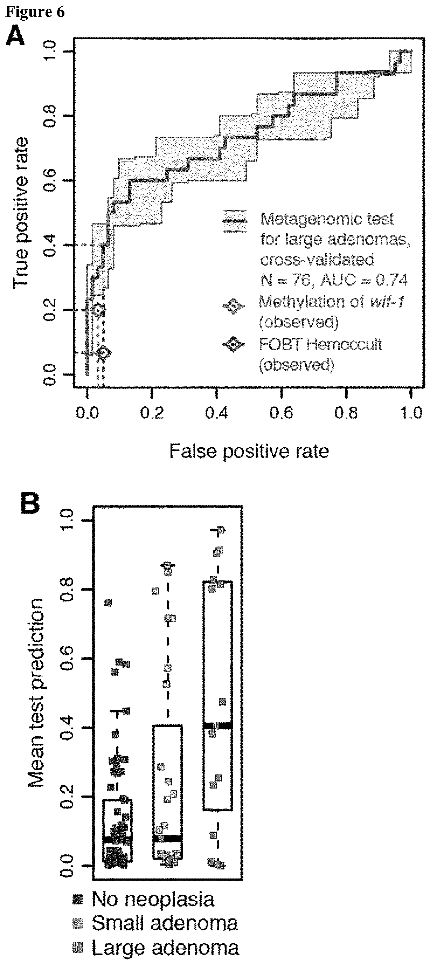

Figure 6 shows the microbial detection of advanced adenomas. (A) A metagenomic classifier was trained to distinguish advanced (large) adenomas (N = 15) from non-neoplastic controls (N = 61, both part of study population F) based on microbial species abundances in feces. Cross-validation accuracy depicted as median ROC curve (ten times resampled ten-fold cross validation on study population F) with grey area indicating the range between the 10th and 90th percentile of ROC curves. Additionally, the accuracy of the *wif-1* methylation test (Lee et al., 2009; Roperch and Sobhani) and the FOBT test is indicated as assessed for the same patients. (B) Application of the classifier from (A) to small adenoma patients (which are not part of the training set) results in intermediate prediction scores which suggests that some of these harbor microbes characteristic of advanced adenomas. Boxplots are as defined in Fig. 5.
Figure 7 shows Metagenomic classifier for the detection of advanced adenomas.
   Relative abundances of gut microbial species that are collectively used as predictors for advanced (large) adenomas are displayed as heatmap in the left panel as fold change over the median relative abundance observed in neoplasia-free controls. This median relative abundance of control samples is indicated to the right of the heatmap. For each adenoma marker species, bar length indicates its mean contribution to adenoma classification (log-odds ratios in multivariate logistic regression, see Online Methods for additional details. Below, the classification score (from cross validation) is shown as gray scale (see key) using colonoscopy results as a ground truth. Displayed alongside are the results of the standard Hemoccult FOBT test routinely applied for CRC screening and an experimental CRC screening test based on methylation of the *wif-1* gene, a *Wnt* pathway member (*35, 36*)

### Examples

### Methods

### Fecal sample collection

Fresh stool samples were collected 2 weeks to 3 days before colonoscopy and in all cases prior to bowel cleanse. Whole fresh stool was collected in sterile boxes, and 10g were frozen at - 20°C within 4 hours and stored. 156 samples were then selected for DNA extraction and shotgun sequencing, among them samples from 53 patients with CRC, 42 adenoma patients and 61 randomly chosen controls. Colonic neoplasia status was determined by colonoscopy. For a subset of 129 patients, 16S rDNA could be amplified from the same DNA extracts and subjected to 16S amplicon sequencing.

### Tissue samples for 16S rRNA gene sequencing

Matched tissue samples (tumor and nearby morphologically healthy mucosa) were collected from 48 patients within ∼30 min after surgical resection, immediately snap frozen and stored at -80°C until DNA extraction and 16S rRNA gene sequencing. Of the 48 patients, 13 patients also provided fecal samples.

### DNA extraction from stool and tissue samples

Genomic DNA was extracted from frozen or RNAlater-preserved fecal samples as previously described using the GNOME® DNA Isolation Kit (MP Biomedicals) with the following minor modifications: cell lysis/denaturation was performed (30 min, 55°C) before protease digestion was carried out overnight (55°C) and RNAse digestion (50 µl, 30 min, 55°C) was performed after mechanical lysis. Tissue samples were thawed carefully, cleaned from remaining feces with sterile Dulbecco's PBS (PAA Laboratories) if necessary and the blades were cleaned with Incidin® Plus (Ecolab) between samples. DNA was extracted from the tissue surfaces using the protocol above. After final precipitation, the DNA was resuspended in TE buffer, and stored at -20°C for further analysis.

### Metagenomic sequencing

Whole genome shotgun sequencing of fecal samples collected was carried out on the Illumina HiSeq 2000/2500 (Illumina, San Diego, USA) platform. All samples were paired-end sequenced with 100 bp read length to a targeted sequencing depth of 5 Gbp.

### 16S rRNA gene sequencing

DNA from 48 tissue sample pairs (tumor and healthy mucosa) and 129 fecal samples was amplified using primers targeting the V4 region of the 16S rRNA gene. PCR was carried out according to the manufacturer's instructions of the Q5 high-fidelity polymerase (New England BioLabs, Ipswich, USA) using barcoded primers (NEXTflex™ 16S V4 Amplicon-Seq Kit, Bioo Scientific, Austin, Texas, USA) at final concentrations of 0.2 µM and an annealing temperature of 56°C for 35 cycles.

PCR products were cleaned up with Agencourt AMPure XP - PCR Purification system (Beckman Coulter, Brea, USA), quantified according to the NEXTflex™ 16S V4 Amplicon-Seq Kit protocol, and multiplexed at equal concentration. Sequencing was performed using a 250 bp paired-end sequencing protocol on the Illumina MiSeq platform (Illumina, San Diego, USA).

### Taxonomic profiling of fecal samples

Using MOCAT (option screen with alignment length cut-off 45 and minimum 97% sequence identity) taxonomic relative abundance profiles were generated by mapping screened high quality reads from each metagenome to a database consisting of 10 universal single-copy marker genes extracted from 3,496 NCBI reference genomes (Mende et al., 2013; Sunagawa *et al.,* 2013). Quantification proceeded in two steps, by first estimating taxonomic abundances from all sequenced DNA fragments (nucleotide counts; each read contributing number of counts equal to its length) that mapped uniquely, and in a second step, nucleotide counts from reads mapping to multiple taxa with the same alignment score were distributed among them proportionally to nucleotide counts originating from reads uniquely mapped to these taxa. Finally, base counts were gene-length normalized (option profiling). Abundance estimates at species level were made based on a recently proposed consistent species-level clustering (Mende et al., 2013), while abundance summarization at higher taxonomic levels was based on the NCBI Taxonomy.

### Taxonomic profiling of tissue and fecal samples using 16S rRNA gene sequences

Raw sequencing data were quality-controlled as described below for the generation of the metagenomic gene catalogue (minimum read length = 45 bp; minimum base quality score = 20). Paired-end reads were merged using the SeqPrep software (J.S. John, GitHub repository: SeqPrep. https://github.com/jstjohn/SeqPrep (2013)) requiring perfect overlap between the high-quality paired-end reads.

After excluding reads shorter than 200 nt or with more than five ambiguous bases, the inventors aligned them to the SILVA 16S reference database of bacterial and archaeal 16S rRNA sequences. These alignments were cropped to only retain the align region spanning V3 to V4 using mothur, version 1.30.2 (Schloss *et al.,* 2009). With the screening and alignment routines the inventors reanalyzed 16S rRNA sequencing reads from (Kostic *et al.,* 2012) to facilitate comparisons across data sets. For further analysis, the inventors only retained sample pairs with at least 1,000 reads aligned in the tumor as well as the normal tissue sample (a requirement met by 79 sample pairs from (Kostic *et al.,* 2012). The boundaries of this core alignment were adjusted to accommodate both the pyrosequencing reads from (Kostic *et al.,* 2012) and the inventors' from the MiSeq platform, by first separately determining the region spanned by 90% of the reads from each sequencing platform, and second by interval merging to cover both of these regions.

For high-resolution taxonomic profiling OTUs from large collections of published 16S rRNA genes were built by including 436,028 sequences from the SILVA database (version 115, (E. Pruesse et al, 2007) and 5,224 extracted from the prokaryotic genome sequences used for taxonomic profiling of metagenomic reads (see above). These were aligned with the same protocol as the 16S reads and cropped to the core alignment region using mothur. Subsequently redundancies were removed by dereplication (mothur's unique.seqs) and clustering at 98% sequence identity using UCLUST (version 6.0.307 with the option -maxrejects 1000 for more accurate matching). As an initial quantification and ordering for the heuristic UCLUST algorithm, the inventors mapped 10,000 reads from each sample against the reference sequences and sorted them according to the number of mapped reads using USEARCH routines (98% ID, best-hit). After clustering reference sequences into 2,460 OTUs (with a minimum size of 10), mapped all 16S Illumina and pyrosequencing reads were mapped against these taking a best-hit approach with a minimum of 98% sequence identity between matches (using USEARCH with default settings).

### Results

### The gut microbiome of the CRC study population

To explore the association of the gut microbiome with colorectal carcinoma (CRC) in non-invasive samples, the inventors analyzed fecal metagenomes from a first population of 156 participants recruited in France (study population F in the following), who underwent colonoscopy to either diagnose colonic neoplasia in the form of adenoma(s) (polyps) or CRC, or to confirm the absence of these in the control group; Carcinomas were further classified according to established staging systems (AJCC and TNM) (O'Connell et al., 2004). The inventors first analyzed global community properties: While CRC-associated dysbiosis did not result in significant changes of microbial community diversity, species or gene richness, the distribution of enterotypes (Arumugam et al., 2011) varied slightly between these groups. Also taxonomic community composition (Kultima et al., 2012) appeared broadly similar at the phylum level, except for gram-negative *Fusobacteria* and *Proteobacteria* with significant increases in CRC patients. However, at the lower taxonomic ranks of genera and species, the inventors detected large and statistically significant differences between CRC and tumor-free controls (the latter group including participants with adenomas). Since alterations appeared most discriminative at the species level, the inventors explored those further.

### Metagenomic classifier for CRC

Although many species are significantly associated with CRC, not a single one differing in abundance, would individually be suitable as a biomarker for an accurate screening assay. The inventors therefore analyzed how well combinations of a few species would be able to distinguish CRC patients from tumor-free controls (including adenoma patients) using a classification methodology based on LASSO penalized linear models (Tibshirani, 1996). The consensus signature, extracted from an ensemble of classifiers that were trained on taxonomic abundance profiles, consisted of 16 species (Fig. 1A).

On average, more than 53% of the total absolute weight of these classification models can be attributed to the abundance differences of only four most discriminative species: the two *Fusobacterium nucleatum* subspecies *animalis* and *vincentii, Porphyromonas asaccharolytica* and *Peptostreptococcus stomatis,* all of which are enriched in CRC (Fig. 1A). In cross validation within study population F, the metagenomic classifiers accurately detected CRC patients: in an evaluation based on receiver operating characteristic (ROC) curves, they achieved a median area under the ROC curve (AUC) of 0.80 (Fig. 1B).

Although it is not yet clear whether and which gut microbiota are causally involved in CRC, recent evidence suggests *Fusobacterium* species (in particular *F. nucleatum*) to be prevalent CRC-associated microbes (Castellarin et al., 2012; Kostic et al., 2012) and to accelerate tumorigenesis (Kostic et al., 2013; Rubinstein et al., 2013). Here, the inventors further refine this to two subspecies of *Fusobacterium nucleatum* (*vincentii* and *animalis*), both of which are distinct enough from *F. nucleatum* subsp. *nucleatum* and to each other to qualify as independent species (Mende et al., 2013). Yet, only a combination of species together (notably involving also *Porphyromonas* spp. and *Peptostreptococcus* spp.) appears to form the basis of reliable CRC detection.

As the age distribution differs between cases and controls in this study population, the inventors ruled out that the metagenomic classifier might exploit potential correlations of microbial abundances with host age rather than CRC. Indeed, the classifier neither showed an increase in false-positive rate nor in sensitivity for older patients. Overall, the accuracy of the metagenomic classifier appears better than that of the Hemoccult FOBT test (Fig. 1B); this test, basically measuring the amount of blood in feces, is routinely used in mass screening for CRC and was applied to study population F prior to colonoscopy (Allison et al., 1996; Faivre et al., 2004; Zavoral et al., 2009). The accuracy of another experimental screening assay based on a different readout, namely the level of gene methylation of the *Wnt* signaling pathway member, *wif-1* (Lee et al., 2009; Roperch and Sobhani), was also matched by the metagenomic test (Fig. 1B).

As the features captured by FOBT and the metagenome appeared to be partially independent (Fig. 1 A), the inventors further evaluated a combination test, which led to a further improvement in accuracy (AUC 0.83).

### Validation of the CRC classifier using patients and controls from different countries

The broad utility of gut microbial markers might be limited by geographical or ethnical particularities or by technical variations in experimental procedures (de Vos and Nieuwdorp, 2013; Sunagawa et al., 2013), illustrated by distinct microbial associations with type 2 diabetes in Chinese and Swedish studies (Karlsson et al., 2013; Qin et al., 2012). The inventors therefore sought to validate the metagenomic CRC classifier also in an independent group of individuals from different countries. To assess whether it maintains high specificity in a large control population, the inventors applied the classifier to five samples from healthy German individuals and 292 published fecal metagenomes from Danish and Spanish individuals who were neither diagnosed with CRC nor inflammatory bowel disease (IBD) (Le Chatelier et al., 2013; Qin et al., 2010) (study population H). At a decision boundary of 0.6 (that is, above this value the classifier predicts CRC, see Fig. 1), the resulting false positive rate (i.e. 1 - specificity) varied slightly from 3.9% to 5.4% between cross validation on study population F and independent validation on study population H, where the true error rate might be slightly overestimated, because absence of CRC has not been confirmed by colonoscopy. To also independently validate the sensitivity of the classifier, the inventors sequenced an additional 38 faecal metagenomes from individuals who were diagnosed with CRC by colonoscopy in a German hospital (study population G). On this data set the sensitivity (i.e. true positive rate) of the metagenomic CRC classifier was 44.7% compared to 47.2% observed in cross-validation. On the combined validation set of study populations F and H, the metagenomic CRC classifier achieved an accuracy of 0.78 AUC, which is very similar to its cross-validation performance (Fig. 1BC). Taken together these results indicate that, despite differences between study populations in nationality and demographics, metagenomic CRC detection is possible with high accuracy, broadly applicable, and robust to minor technical variation, all prerequisites for screening.

In order to estimate how much classification accuracy would improve with a larger study population, the inventors included the 38 CRC patients from study populations G into study population F and applied the same modelling approach to train and cross-validate a more comprehensive classifier. Its cross-validation accuracy increased to 0.84 AUC (Fig. 1C), which appears superior to currently available FOBT variants (Allison et al., 1996; Faivre et al., 2004), although this would have to be confirmed in larger study populations.

### Detection of CRC in different stages of tumor progression

As an instrument for reducing CRC mortality, patient screening is most effective if cancer is diagnosed early, before the tumor has metastasized to nearby lymph nodes or distant tissues (O'Connell et al., 2004). To rule out that the inventor's metagenomic classifier is biased towards late-stage carcinomas, for which changes in the colon environment and its microbiota might be more pronounced than in earlier stages of tumor development, the inventors compared the sensitivity for localized CRC (AJCC stages 0, I, and II) to that for metastasized tumors (stages III and IV). The inventors found that early CRC was detected with comparable or higher sensitivity as metastasized tumors in both study populations F and G (Fig. 1D), confirming the potential of microbial markers for early detection of CRC. Additionally, the inventors explored how the abundances of the four most discriminative CRC-markers correlated with CRC progression from early neoplastic growth to late-stage metastasizing tumors (with samples stratified into four groups of neoplasia-free participants, participants with adenoma, stage 0 / I / II, and stage III / IV CRC patients) (Fig. 1D). All of these markers showed a significant correlation (Spearman correlation |rho| between 0.23 and 0.43, all p values < 0.001), and a strong enrichment in early-stage CRC patients compared to controls was evident for both *Fusobacterium nucleatum* sub spp. and *Peptostreptococcus stomatis* (Fig. 1E). Likewise a depletion of some microbiota that were negatively associated with CRC, such as *Eubacterium* spp. And *Ruminococcus* spp., was noticeable in early-stage patients already.

### CRC marker species in inflammatory bowel disease

The observed associations of microbiota with CRC alone do not reveal how specific they are to this particular disease. To exclude that there might be a more general dysbiosis common to multiple disease conditions (e.g. due to inflammation), the inventors applied the inventor's classifier to 25 recently published metagenomes from inflammatory bowel disease (IBD) patients (21 Ulcerative colitis (UC) and 4 Crohn's disease (CD) patients) (Qin et al., 2010). Although small sample size precludes precise estimates, the inventor's results indicate a moderate increase of false-positive predictions to 8% in these patients, which is about twice the rate seen in other controls (Fig. 2A). This may reflect common alterations in the microbiota in these diseases, which is consistent with IBD patients being at greater risk of developing CRC (Bernstein et al., 2001). To explore this further, the inventors monitored the four most discriminative CRC marker species for significant changes in abundance in IBD patients relative to controls (Fig. 2B). Although higher prevalence of *Fusobacterium* species in IBD patients has been reported (Strauss et al., 2011), the inventors did not observe a significant increase for the two CRC marker species from this genus in IBD patients. For the most discriminative marker species that the inventor's model positively associated with CRC the inventors found significantly higher levels in CRC compared to IBD patients, indicating that these markers are specific to CRC (Fig 2B). In contrast, *Eubacterium eligens,* which is negatively associated with CRC, showed an even stronger decrease in IBD patients than in CRC consistent with a study that reported reduced colonization of the intestinal mucus with *Eubacterium* species in IBD (Swidsinski et al., 2005). These results suggest that the metagenomic classifier is quite specific for CRC and only modestly influenced by changes in the microbiota that are due to inflammation.

### Fecal CRC markers reflect enrichments in tumor biopsies

In order to make use of functional data extracted from the metagenome, fecal samples would need to reflect, at least partially, the microbial composition in the tumor environment. However, profiling colonic tissue samples with shotgun metagenomic sequencing is still ineffective due to excessive contamination with human DNA (Castellarin et al., 2012; Kostic et al., 2012). As an alternative, targeted sequencing of prokaryotic 16S rRNA gene fragments from tumor biopsies allows for taxonomic abundance estimation and identification of enriched microbes compared to nearby intact mucosa. For this, the inventors newly sequenced 48 tumor-normal tissue pairs (13 from patients that were also part of study population G) and reanalyzed 79 such deeply sequenced pairs from a published study with US American and Spanish patients (Kostic et al., 2012). To be able to distinguish relevant differences between the microbial communities at the tumor site and in stool from technical disparities due to different sample processing methods, the inventors collected a third data set of fecal samples (part of study population F) subjected to 16S amplicon sequencing. To profile the taxonomic composition of 16s rDNA samples, the inventors constructed operational taxomomic units (OTUs, 98% sequence identity) from comprehensive databases of published 16S sequences and mapped 16S reads against these. To facilitate comparisons across data sets, OTUs were further matched to the marker species from the fecal metagenomic signature of study population F (using a best-hit approach based on the respective 16S gene fragments). When comparing relative abundances between data sets (Fig. 3), both fecal CRC marker species from the *Fusobacterium* genus showed a consistent enrichment at the tumor site, as was expected from previous studies (Castellarin et al., 2012; Kostic et al., 2012; Marchesi et al., 2011; Warren et al., 2013). Higher detection rates (prevalences) were observed in the tumor microenvironment than in feces (Fig. 3) suggesting that deep sequencing of fecal material is necessary to overcome this dilution effect. However in the datasets compared here, abundance differences between CRC patients and tumor-free controls were at least as significant as between tumor and normal tissue. The inventors further observed increased abundance of *Peptostreptococcus stomatis* in CRC consistent with biopsies, but this trend was only significant in the published data set (Kostic et al., 2012). Where comparability could be established across sequencing technologies, most metagenomic marker species with significantly lower abundance in study population F also showed significant abundance changes in normal tissue compared to tumors, as was the case for *Eubacterium* spp. and *Streptococcus salivarius.* These results indicate that similar trends for the relative abundances of marker species between fecal and biopsy samples from CRC patients are detectable despite the apparent differences in patient nationality, sample origin, experimental techniques, and analysis methodology. The inventors furthermore verified that the similarity between tumor-associated microbiota and enrichments in fecal samples from CRC patients is not confined to the marker species only, but also manifests as a dominant trend in principal component analysis (PCA).

All this shows that fecal readouts may also allow for inferences of the metabolic and functional potential of the colonic microbiome in the tumor environment.

### Functional changes in the CRC-associated fecal microbiome

To characterize microbial gene functions and how these differ between CRC patients and tumor-free participants, the inventors quantified the relative abundances of prokaryotic KEGG (Kyoto Encyclopedia of Genes and Genomes) modules (Kanehisa et al., 2008) in each metagenome of study population F. To investigate carbohydrate utilization preferences of the microbiota (El Kaoutari et al., 2013; Koropatkin et al., 2012; Sonnenburg et al., 2005), the inventors additionally used prokaryotic families of carbohydrate-active enzymes from the CA-Zy database (Cantarel et al., 2009) to annotate metagenomes. As a result, the inventors found 24 KEGG modules and 20 CAZy families to significantly differ in abundance in CRC patients with a fold change >1.33 (Fig. 4).

Analysis of the functions that significantly differed between healthy participants and cancer patients revealed a global metabolic shift from predominant utilization of dietary fiber in the tumor-free colon to mostly host-derived energy sources in CRC (Fig. 4B). In healthy gut metagenomes, only fiber-degrading enzymes and fiber binding domains were enriched, whereas in CRC patients the microbiota appeared to exploit growth substrates derived from host cells to a much larger extent (5-fold enrichment of host glycans in CRC, p = 0.01, Fisher test, Fig 4B). Thus, the inventors hypothesize that an increased degradation of host glycans might be related to the etiology of CRC. However, the inventors cannot rule out that this shift from dietary fiber to animal glycans might reflect different dietary preferences between CRC patients and controls in the inventor's study population, as epidemiological data indicate that a fiber-rich diet reduces cancer risk, while a diet rich in meat and animal fat increases it (Bingham et al., 2003; Greer and O'Keefe, 2011; World Cancer Research Fund / American Institute for Cancer Research, 2011). Hence it is also conceivable that the microbiota might act as a mediator of these dietary effects (see e.g. Claesson et al., 2012; Le Chatelier et al., 2013; Turnbaugh et al., 2009). Host cell-wall carbohydrates, such as mucins, whose utilization is enriched in the CRC metagenomes of study population F, have been established as an important energy source for commensal microbiota of the healthy gut (Bergstrom and Xia, 2013; Koropatkin et al., 2012; Sonnenburg et al., 2005). However, compromised integrity of the inner mucus layer that functions to shield the epithelium from luminal bacteria might accelerate the progression of CRC and inflammatory bowel disease: In animal models, mucin gene defects can lead to intestinal cancers or microbiota-dependent acute colitis (Bergstrom and Xia, 2013; Fu et al., 2011; Velcich et al., 2002), and in IBD patients increased mucolytic activity has been reported for mucus-associated bacteria (Png et al., 2010). It is therefore conceivable that degrading the mucus barrier might be a strategy that is adopted by adhesive and/or invasive pathogens such as *Fusobacterium* spp. to reach the epithelial cells (Dharmani et al., 2011; McGuckin et al., 2011; Rubinstein et al., 2013).

Some host-cell derived metabolites are more abundant in the tumor environment, for instance amino acids, of which elevated levels have been measured in CRC patients by metabolomics (Weir et al., 2013). The inventor's data showed an increased capacity of the CRC-associated microbiota for uptake and metabolism of some amino acids via the putrefaction pathway (Fig. 4A). Their degradation products include polyamines (like putrescine), which at increased intracellular levels promote tumor development (Gerner and Meyskens, 2004). That enterotoxigenic *Bacteroides fragilis* (BFTs) can exploit this by stimulating the endogenous polyamine catabolism in colonic epithelial cells was recently shown (Goodwin et al., 2011).

Although the inventor's functional analysis cannot reveal whether the observed enrichment of putrefaction in the CRC microbiome is a consequence of tumor metabolism or whether it contributes causally to tumor progression, it provides additional evidence for the "oral microbiome hypothesis" (Warren et al., 2013). Previously, it has been noted that several CRC-associated bacteria, e.g. *Fusobacterium* spp., were first described as oral pathogens (Warren et al., 2013), which is also the case for *Peptostreptococcus stomatis* (Downes and Wade, 2006). Similarly, putrescine/spermidine metabolism has been described as a core trait of the oral microbiota (Abubucker et al., 2012; Shafquat et al., 2014).

Concomitant with the metabolic shift in the CRC microbiome, the inventors observed an expanded repertoire of proinflammatory and pathogenicity processes, most notably increased potential for lipopolysaccharide (LPS) metabolism (Fig. 4), which is consistent with a CRC-associated expansion of Gram-negative bacteria that bear LPS antigens on their outer membranes. Through binding to Toll-like receptor 4 (TLR4) in epithelial cells, LPS triggers an inflammatory signaling cascade, which in turn, could promote inflammation-induced carcinogenesis (Cario et al., 2000; Tang et al., 2010) and even metastasis, as has been demonstrated in mice (Hsu et al., 2011). An enrichment of hemolysin transport, RTX toxin transport and type II secretion systems in CRC metagenomes might hint at an increase of pathogenicity processes encoded in the genomes of Gram-negative bacteria. To examine virulence factors and secreted toxins whose potential roles in the etiology of CRC were discussed before (Boleij and Tjalsma, 2012), the inventors profiled a manually curated list of 15 toxin families and virulence factors in the inventor's metagenomic data.

However, most of these were either not detectable in fecal metagenomes or not enriched in CRC patients, except for *Fusobacterium* adhesin (*fadA*), which was recently shown to be required for its invasiveness and tumorigenesis (Rubinstein et al., 2013; Strauss et al., 2011).

Analyzed here for the first time, the complex functional alterations in the microbiome of CRC patients appear to occur gradually during CRC progression from precancerous stages to metastasized carcinomas (Fig. 4). To directly assess this correlation, the inventors applied principal component analysis (PCA) to significantly altered KEGG modules and CAZY families (as shown in Fig. 4, using the same grouping of patients by CRC stage as above). Indeed, the first principal component capturing the dominant source of functional variation between CRC patients and controls (PC1, explaining 43% of total variance) strongly correlated with neoplasia progression (Spearman's rho > 0.45, p < 1E-8, Fig. 5), indicating that some functional changes in the microbiota are already detectable in early stages of neoplastic growth.

### Detection of precancerous lesions

As both genes and species markers indicate microbiota changes already during early stages of neoplastic growth (Fig. 1E, 4, 5), the inventors also sought to predict pre-cancers, in particular advanced adenomas. As a proof of principle, the inventors trained a classifier to distinguish between taxonomic abundance profiles derived from 15 advanced adenoma patients and 51 from neoplasia-free controls (part of study population F) using the same methodology as for CRC detection. Indeed, this classifier achieved an accuracy of 0.74 AUC, corresponding to an increase of sensitivity for advanced adenoma detection over FOBT from <10% to ∼40% (Fig. 6 and Figure 7); low sensitivity of the FOBT for pre-cancers has been noted in larger evaluations before (e.g. Allison et al., 1996).

### Literature as cited

Abubucker, S., Segata, N., Goll, J., Schubert, A.M., Izard, J., Cantarel, B.L., Rodriguez-Mueller, B., Zucker, J., Thiagarajan, M., Henrissat, B., et al. (2012). Metabolic reconstruction for metagenomic data and its application to the human microbiome. PLoS computational biology 8, e1002358.
Allison, J.E., Sakoda, L.C., Levin, T.R., Tucker, J.P., Tekawa, I.S., Cuff, T., Pauly, M.P., Shlager, L., Palitz, A.M., Zhao, W.K., et al. (2007). Screening for colorectal neoplasms with new fecal occult blood tests: update on performance characteristics. J. Natl. Cancer Inst. 99, 1462-1470.
Allison, J.E., Tekawa, I.S., Ransom, L.J., and Adrain, A.L. (1996). A comparison of fecal occult-blood tests for colorectal-cancer screening. N. Engl. J. Med. 334, 155-159.
Altschul, S.F., Gish, W., Miller, W., Myers, E.W., and Lipman, D.J. (1990). Basic local alignment search tool. J. Mol. Biol. 215, 403-410.
Arthur, J.C., Perez-Chanona, E., Muhlbauer, M., Tomkovich, S., Uronis, J.M., Fan, T.J., Campbell, B.J., Abujamel, T., Dogan, B., Rogers, A.B., et al. (2012). Intestinal inflammation targets cancer-inducing activity of the microbiota. Science 338, 120-123.
Arumugam, M., Harrington, E.D., Foerstner, K.U., Raes, J., and Bork, P. (2010). Smash-Community: a metagenomic annotation and analysis tool. Bioinformatics 26, 2977-2978.
Arumugam, M., Raes, J., Pelletier, E., Le Paslier, D., Yamada, T., Mende, D.R., Fernandes, G.R., Tap, J., Bruls, T., Batto, J.M., et al. (2011). Enterotypes of the human gut microbiome. Nature 473, 174-180.
Bergstrom, K.S., and Xia, L. (2013). Mucin-type O-glycans and their roles in intestinal homeostasis. Glycobiology 23,1026-1037.
Bernstein, C.N., Blanchard, J.F., Kliewer, E., and Wajda, A. (2001). Cancer risk in patients with inflammatory bowel disease: a population-based study. Cancer 91, 854-862.
Bingham, S.A., Day, N.E., Luben, R., Ferrari, P., Slimani, N., Norat, T., Clavel-Chapelon, F., Kesse, E., Nieters, A., Boeing, H., et al. (2003). Dietary fibre in food and protection against colorectal cancer in the European Prospective Investigation into Cancer and Nutrition (EPIC): an observational study. Lancet 361, 1496-1501.
Boleij, A., and Tjalsma, H. (2012). Gut bacteria in health and disease: a survey on the interface between intestinal microbiology and colorectal cancer. Biol. Rev. Camb. Philos. Soc. 87, 701-730.
Cancer Genome Atlas Network (2012). Comprehensive molecular characterization of human colon and rectal cancer. Nature 487, 330-337.
Cantarel, B.L., Coutinho, P.M., Rancurel, C., Bernard, T., Lombard, V., and Henrissat, B. (2009). The Carbohydrate-Active EnZymes database (CAZy): an expert resource for Gly-cogenomics. Nucleic Acids Res. 37, D233-238.
Cantarel, B.L., Lombard, V., and Henrissat, B. (2012). Complex carbohydrate utilization by the healthy human microbiome. PLoS One 7, e28742.
Cario, E., Rosenberg, I.M., Brandwein, S.L., Beck, P.L., Reinecker, H.C., and Podolsky, D.K. (2000). Lipopolysaccharide activates distinct signaling pathways in intestinal epithelial cell lines expressing Tolllike receptors. J. Immunol. 164, 966-972.
Castellarin, M., Warren, R.L., Freeman, J.D., Dreolini, L., Krzywinski, M., Strauss, J., Barnes, R., Watson, P., Allen-Vercoe, E., Moore, R.A., et al. (2012). Fusobacterium nucleatum infection is prevalent in human colorectal carcinoma. Genome Res. 22, 299-306. Claesson, M.J., Jeffery, I.B., Conde, S., Power, S.E., O'Connor, E.M., Cusack, S., Harris, H.M., Coakley, M., Lakshminarayanan, B., O'Sullivan, O., et al. (2012). Gut microbiota composition correlates with diet and health in the elderly. Nature 488, 178-184.
Cross, A.J., Ferrucci, L.M., Risch, A., Graubard, B.I., Ward, M.H., Park, Y., Hollenbeck, A.R., Schatzkin, A., and Sinha, R. (2010). A large prospective study of meat consumption and colorectal cancer risk: an investigation of potential mechanisms underlying this association. Cancer Res. 70, 2406-2414.
Cuevas-Ramos, G., Petit, C.R., Marcq, I., Boury, M., Oswald, E., and Nougayrede, J.P. (2010). Escherichia coli induces DNA damage in vivo and triggers genomic instability in mammalian cells. Proceedings of the National Academy of Sciences of the United States of America 107, 11537-11542.
de Martel, C., Ferlay, J., Franceschi, S., Vignat, J., Bray, F., Forman, D., and Plummer, M. (2012). Global burden of cancers attributable to infections in 2008: a review and synthetic analysis. Lancet Oncol. 13, 607-615.
de Vos, W.M., and Nieuwdorp, M. (2013). Genomics: A gut prediction. Nature 498, 48-49.
Dharmani, P., Strauss, J., Ambrose, C., Allen-Vercoe, E., and Chadee, K. (2011). Fusobacterium nucleatum infection of colonic cells stimulates MUC2 mucin and tumor necrosis factor alpha. Infect. Immun. 79, 2597-2607.
Downes, J., and Wade, W.G. (2006). Peptostreptococcus stomatis sp. nov., isolated from the human oral cavity. Int. J. Syst. Evol. Microbiol. 56, 751-754.
Dutilh, B.E., Backus, L., van Hijum, S.A., and Tjalsma, H. (2013). Screening metatranscrip-tomes for toxin genes as functional drivers of human colorectal cancer. Best Pract. Res. Clin. Gastroenterol. 27, 85-99.
Eddy, S.R. (2011). Accelerated Profile HMM Searches. PLoS Comput. Biol. 7, e1002195. Edgar, R.C. (2010). Search and clustering orders of magnitude faster than BLAST. Bioinformatics 26, 2460-2461.
El Kaoutari, A., Armougom, F., Gordon, J.I., Raoult, D., and Henrissat, B. (2013). The abundance and variety of carbohydrate-active enzymes in the human gut microbiota. Nat. Rev. Microbiol. 11, 497-504.
Faivre, J., Dancourt, V., Lejeune, C., Tazi, M.A., Lamour, J., Gerard, D., Dassonville, F., and Bonithon-Kopp, C. (2004). Reduction in colorectal cancer mortality by fecal occult blood screening in a French controlled study. Gastroenterology 126, 1674-1680.
Fan, R.E., Chang, K.W., Hsieh, C.J., Wang, X.R., and Lin, C.J. (2008). LIBLINEAR: A library for large linear classification. JMLR 9, 1871-1874.
Fasano, A. (2002). Toxins and the gut: role in human disease. Gut 50 III9-14.
Fisher, S., Barry, A., Abreu, J., Minie, B., Nolan, J., Delorey, T.M., Young, G., Fennell, T.J., Allen, A., Ambrogio, L., et al. (2011). A scalable, fully automated process for construction of sequence-ready human exome targeted capture libraries. Genome Biol. 12, R1.
Flanagan, L., Schmid, J., Ebert, M., Soucek, P., Kunicka, T., Liska, V., Bruha, J., Neary, P., Dezeeuw, N., Tommasino, M., et al. (2014). Fusobacterium nucleatum associates with stages of colorectal neoplasia development, colorectal cancer and disease outcome. European journal of clinical microbiology & infectious diseases : official publication of the European Society of Clinical Microbiology.
Fu, J., Wei, B., Wen, T., Johansson, M.E., Liu, X., Bradford, E., Thomsson, K.A., McGee, S., Mansour, L., Tong, M., et al. (2011). Loss of intestinal core 1-derived O-glycans causes spontaneous colitis in mice. J. Clin. Invest. 121, 1657-1666.
Furet, J.P., Firmesse, O., Gourmelon, M., Bridonneau, C., Tap, J., Mondot, S., Dore, J., and Corthier, G. (2009). Comparative assessment of human and farm animal faecal microbiota using real-time quantitative PCR. FEMS Microbiol. Ecol. 68, 351-362.
Gerner, E.W., and Meyskens, F.L., Jr. (2004). Polyamines and cancer: old molecules, new understanding. Nat. Rev. Cancer 4, 781-792.
GitHub (2013). SeqPrep. https://github.com/jstjohn/SeqPrep.
Goodwin, A.C., Destefano Shields, C.E., Wu, S., Huso, D.L., Wu, X., Murray-Stewart, T.R., Hacker-Prietz, A., Rabizadeh, S., Woster, P.M., Sears, C.L., et al. (2011). Polyamine catabolism contributes to enterotoxigenic Bacteroides fragilis-induced colon tumorigenesis. Proceedings of the National Academy of Sciences of the United States of America 108, 15354-15359.
Greer, J.B., and O'Keefe, S.J. (2011). Microbial induction of immunity, inflammation, and cancer. Front. Physiol. 1, 168.
Hol, L., van Leerdam, M.E., van Ballegooijen, M., van Vuuren, A.J., van Dekken, H., Reijerink, J.C., van der Togt, A.C., Habbema, J.D., and Kuipers, E.J. (2010). Screening for colorectal cancer: randomised trial comparing guaiac-based and immunochemical faecal occult blood testing and flexible sigmoidoscopy. Gut 59, 62-68.
Hsu, R.Y., Chan, C.H., Spicer, J.D., Rousseau, M.C., Giannias, B., Rousseau, S., and Ferri, L.E. (2011). LPS-induced TLR4 signaling in human colorectal cancer cells increases betal integrin-mediated cell adhesion and liver metastasis. Cancer Res. 71, 1989-1998.
Human Microbiome Project Consortium (2012). Structure, function and diversity of the healthy human microbiome. Nature 486, 207-214.
Jemal, A., Bray, F., Center, M.M., Ferlay, J., Ward, E., and Forman, D. (2011). Global cancer statistics. CA Cancer J. Clin. 61, 69-90.
Kanehisa, M., Araki, M., Goto, S., Hattori, M., Hirakawa, M., Itoh, M., Katayama, T., Kawashima, S., Okuda, S., Tokimatsu, T., et al. (2008). KEGG for linking genomes to life and the environment. Nucleic Acids Res. 36, D480-484.
Karlsson, F.H., Tremaroli, V., Nookaew, I., Bergstrom, G., Behre, C.J., Fagerberg, B., Nielsen, J., and Backhed, F. (2013). Gut metagenome in European women with normal, impaired and diabetic glucose control. Nature 498, 99-103.
Knights, D., Parfrey, L.W., Zaneveld, J., Lozupone, C., and Knight, R. (2011). Human-associated microbial signatures: examining their predictive value. Cell Host Microbe 10, 292-296.
Koropatkin, N.M., Cameron, E.A., and Martens, E.C. (2012). How glycan metabolism shapes the human gut microbiota. Nat. Rev. Microbiol. 10, 323-335.
Kostic, A.D., Chun, E., Robertson, L., Glickman, J.N., Gallini, C.A., Michaud, M., Clancy, T.E., Chung, D.C., Lochhead, P., Hold, G.L., et al. (2013). Fusobacterium nucleatum potentiates intestinal tumorigenesis and modulates the tumor-immune microenvironment. Cell Host Microbe 14, 207-215.
Kostic, A.D., Gevers, D., Pedamallu, C.S., Michaud, M., Duke, F., Earl, A.M., Ojesina, A.I., Jung, J., Bass, A.J., Tabernero, J., et al. (2012). Genomic analysis identifies association of Fusobacterium with colorectal carcinoma. Genome Res. 22, 292-298.
Kultima, J.R., Sunagawa, S., Li, J., Chen, W., Chen, H., Mende, D.R., Arumugam, M., Pan, Q., Liu, B., Qin, J., et al. (2012). MOCAT: a metagenomics assembly and gene prediction toolkit. PLoS One 7, e47656.
Le Chatelier, E., Nielsen, T., Qin, J., Prifti, E., Hildebrand, F., Falony, G., Almeida, M., Arumugam, M., Batto, J.M., Kennedy, S., et al. (2013). Richness of human gut microbiome correlates with metabolic markers. Nature 500, 541-546.
Lee, B.B., Lee, E.J., Jung, E.H., Chun, H.K., Chang, D.K., Song, S.Y., Park, J., and Kim, D.H. (2009). Aberrant methylation of APC, MGMT, RASSF2A, and Wif-1 genes in plasma as a biomarker for early detection of colorectal cancer. Clin. Cancer. Res. 15, 6185-6191.
Levin, B., Lieberman, D.A., McFarland, B., Smith, R.A., Brooks, D., Andrews, K.S., Dash, C., Giardiello, F.M., Glick, S., Levin, T.R., et al. (2008). Screening and surveillance for the early detection of colorectal cancer and adenomatous polyps, 2008: a joint guideline from the American Cancer Society, the US Multi-Society Task Force on Colorectal Cancer, and the American College of Radiology. CA Cancer J. Clin. 58, 130-160.
Li, R., Li, Y., Kristiansen, K., and Wang, J. (2008). SOAP: short oligonucleotide alignment program. Bioinformatics 24, 713-714.
Li, W., and Godzik, A. (2006). Cd-hit: a fast program for clustering and comparing large sets of protein or nucleotide sequences. Bioinformatics 22, 1658-1659.
Lichtenstein, P., Holm, N.V., Verkasalo, P.K., Iliadou, A., Kaprio, J., Koskenvuo, M., Pukkala, E., Skytthe, A., and Hemminki, K. (2000). Environmental and heritable factors in the causation of cancer--analyses of cohorts of twins from Sweden, Denmark, and Finland. N. Engl. J. Med. 343, 78-85.
Marchesi, J.R., Dutilh, B.E., Hall, N., Peters, W.H., Roelofs, R., Boleij, A., and Tjalsma, H. (2011). Towards the human colorectal cancer microbiome. PloS one 6, e20447.
McCoy, A.N., Araujo-Perez, F., Azcarate-Peril, A., Yeh, J.J., Sandler, R.S., and Keku, T.O. (2013). Fusobacterium is associated with colorectal adenomas. PloS one 8, e53653.
McGuckin, M.A., Linden, S.K., Sutton, P., and Florin, T.H. (2011). Mucin dynamics and enteric pathogens. Nat. Rev. Microbiol. 9, 265-278.
Mende, D.R., Sunagawa, S., Zeller, G., and Bork, P. (2013). Accurate and universal delineation of prokaryotic species. Nat. Methods 10, 881-884.
O'Connell, J.B., Maggard, M.A., and Ko, C.Y. (2004). Colon cancer survival rates with the new American Joint Committee on Cancer sixth edition staging. J. Natl. Cancer Inst. 96, 1420-1425.
Oksanen, J., Blanchet, F.G., Kindt, R., Legendre, P., Minchin, P.R., O'Hara, R.B., Simpson, G.L., Solymos, P., Stevens, M.H.H., and Wagner, H. (2013). vegan: Community Ecology Package. http://cran.rproject.org/web/packages/vegan/index.html.
Papa, E., Docktor, M., Smillie, C., Weber, S., Preheim, S.P., Gevers, D., Giannoukos, G., Ciulla, D., Tabbaa, D., Ingram, J., et al. (2012). Non-invasive mapping of the gastrointestinal microbiota identifies children with inflammatory bowel disease. PLoS One 7, e39242.
Png, C.W., Linden, S.K., Gilshenan, K.S., Zoetendal, E.G., McSweeney, C.S., Sly, L.I., McGuckin, M.A., and Florin, T.H. (2010). Mucolytic bacteria with increased prevalence in IBD mucosa augment in vitro utilization of mucin by other bacteria. Am. J. Gastroenterol. 105, 2420-2428.
Pritchard, C.C., and Grady, W.M. (2011). Colorectal cancer molecular biology moves into clinical practice. Gut 60, 116-129.
Pruesse, E., Quast, C., Knittel, K., Fuchs, B.M., Ludwig, W., Peplies, J., and Glockner, F.O. (2007). SILVA: a comprehensive online resource for quality checked and aligned ribosomal RNA sequence data compatible with ARB. Nucleic Acids Res. 35, 7188-7196.
Qin, J., Li, R., Raes, J., Arumugam, M., Burgdorf, K.S., Manichanh, C., Nielsen, T., Pons, N., Levenez, F., Yamada, T., et al. (2010). A human gut microbial gene catalogue established by metagenomic sequencing. Nature 464, 59-65.
Qin, J., Li, Y., Cai, Z., Li, S., Zhu, J., Zhang, F., Liang, S., Zhang, W., Guan, Y., Shen, D., et al. (2012). A metagenome-wide association study of gut microbiota in type 2 diabetes. Nature 490, 55-60.
R Core Team (2013). R: A Language and Environment for Statistical Computing. http://www.Rproject.org.
Roperch, J.P., and Sobhani, I. European patent EP2635705 (A1) / US patent 20110129840 A1.
Rubinstein, M.R., Wang, X., Liu, W., Hao, Y., Cai, G., and Han, Y.W. (2013). Fusobacterium nucleatum promotes colorectal carcinogenesis by modulating E-cadherin/beta-catenin signaling via its FadA adhesin. Cell Host Microbe 14, 195-206.
Schloss, P.D., Westcott, S.L., Ryabin, T., Hall, J.R., Hartmann, M., Hollister, E.B., Lesniew-ski, R.A., Oakley, B.B., Parks, D.H., Robinson, C.J., et al. (2009). Introducing mothur: open-source, platform independent, community-supported software for describing and comparing microbial communities. Appl. Environ. Microbiol. 75, 7537-7541.
Shafquat, A., Joice, R., Simmons, S.L., and Huttenhower, C. (2014). Functional and phylogenetic assembly of microbial communities in the human microbiome. Trends in microbiology.
Sievers, F., Wilm, A., Dineen, D., Gibson, T.J., Karplus, K., Li, W., Lopez, R., McWilliam, H., Remmert, M., Soding, J., et al. (2011). Fast, scalable generation of high-quality protein multiple sequence alignments using Clustal Omega. Mol. Syst. Biol. 7, 539.
Smialowski, P., Frishman, D., and Kramer, S. (2010). Pitfalls of supervised feature selection. Bioinformatics 26, 440-443.
Sobhani, I. (2013). Validation of an Alternative Biological Test to Increase the Detection Sensitivity of a Colon Tumour (VATNIMAD). http://clinicaltrials.gov/ct2/show/study/NCT01270360.
Sobhani, I., Tap, J., Roudot-Thoraval, F., Roperch, J.P., Letulle, S., Langella, P., Corthier, G., Tran Van Nhieu, J., and Furet, J.P. (2011). Microbial dysbiosis in colorectal cancer (CRC) patients. PLoS One 6, el6393.
Sonnenburg, J.L., Xu, J., Leip, D.D., Chen, C.H., Westover, B.P., Weatherford, J., Buhler, J.D., and Gordon, J.I. (2005). Glycan foraging in vivo by an intestine-adapted bacterial symbiont. Science 307, 1955-1959.
Strauss, J., Kaplan, G.G., Beck, P.L., Rioux, K., Panaccione, R., Devinney, R., Lynch, T., and Allen-Vercoe, E. (2011). Invasive potential of gut mucosa-derived Fusobacterium nucleatum positively correlates with IBD status of the host. Inflamm. Bowel Dis. 17, 1971-1978.
Sunagawa, S., Mende, D.R., Zeller, G., Izquierdo-Carrasco, F., Berger, S.A., Kultima, J.R., Coelho, L.P., Arumugam, M., Tap, J., Nielsen, H.B., et al. (2013). Metagenomic species profiling using universal phylogenetic marker genes. Nat. Methods.
Swidsinski, A., Weber, J., Loening-Baucke, V., Hale, L.P., and Lochs, H. (2005). Spatial organization and composition of the mucosal flora in patients with inflammatory bowel disease. J. Clin. Microbiol. 43, 3380-3389.
Tahara, T., Yamamoto, E., Suzuki, H., Maruyama, R., Chung, W., Garriga, J., Jelinek, J., Yamano, H.O., Sugai, T., An, B., et al. (2014). Fusobacterium in colonic flora and molecular features of colorectal carcinoma. Cancer Research.
Tang, X.Y., Zhu, Y.Q., Wei, B., and Wang, H. (2010). Expression and functional research of TLR4 in human colon carcinoma. Am. J. Med. Sci. 339, 319-326.
Tibshirani, R. (1996). Regression shinkage and selection via the Lasso. J. R. Statist. Soc. B 58, 267-288.
Toprak, N.U., Yagci, A., Gulluoglu, B.M., Akin, M.L., Demirkalem, P., Celenk, T., and Soyletir, G. (2006). A possible role of Bacteroides fragilis enterotoxin in the aetiology of colorectal cancer. Clin. Microbiol. Infect. 12, 782-786.
Trosset, M.W., and Priebe, C.E. (2006). The out-of-sample problem for classical multidimensional scaling. Comput. Stat. Data Anal. 52, 4635-4642.
Turnbaugh, P.J., Hamady, M., Yatsunenko, T., Cantarel, B.L., Duncan, A., Ley, R.E., Sogin, M.L., Jones, W.J., Roe, B.A., Affourtit, J.P., et al. (2009). A core gut microbiome in obese and lean twins. Nature 457, 480-484.
van Bueren, A.L. (assessed 28 October 2013). Carbohydrate-binding modules. http://www.cazypedia.org/index.php?title=Carbohydrate-binding modules&oldid=9411.
Velcich, A., Yang, W., Heyer, J., Fragale, A., Nicholas, C., Viani, S., Kucherlapati, R., Lip-kin, M., Yang, K., and Augenlicht, L. (2002). Colorectal cancer in mice genetically deficient in the mucin Muc2. Science 295, 1726-1729.
Vogelstein, B., Papadopoulos, N., Velculescu, V.E., Zhou, S., Diaz, L.A., Jr., and Kinzler, K.W. (2013). Cancer genome landscapes. Science 339, 1546-1558.
Wang, Q., Garrity, G.M., Tiedje, J.M., and Cole, J.R. (2007). Naive Bayesian classifier for rapid assignment of rRNA sequences into the new bacterial taxonomy. Appl. Environ. Microbiol. 73, 5261-5267.
Warren, R.L., Freeman, D.J., Pleasance, S., Watson, P., Moore, R.A., Cochrane, K., Allen-Vercoe, E., and Holt, R.A. (2013). Co-occurrence of anaerobic bacteria in colorectal carcinomas. Microbiome 1.
Weir, T.L., Manter, D.K., Sheflin, A.M., Barnett, B.A., Heuberger, A.L., and Ryan, E.P. (2013). Stool microbiome and metabolome differences between colorectal cancer patients and healthy adults. PLoS One 8, e70803.
World Cancer Research Fund / American Institute for Cancer Research (2011). Continuous Update Project Report. Food, Nutrition, Physical Activity, and the Prevention of Colorectal Cancer.

## Claims

1. A method for diagnosing colorectal adenomas and/or colorectal cancer (CRC), comprising the steps of
a) obtaining a fecal sample comprising the gut microbiome from a human patient to be diagnosed,
b) detecting the fecal metagenome in said sample comprising the species
i) *Fusobacterium nucleatum* subsp. *vincentii, Fusobacterium nucleatum* subsp. *animalis, Porphyromonas asaccharolytica,* and *Peptostreptococcus stomatis,* and/or
ii) *Enterobacteriaceae bacterium* 9_2_54FAA, *Acidaminococcus intestini, Bacteroides stercoris,* and *Ruminococcaceae bacterium* D16, and
c) concluding on colorectal adenomas and/or CRC in said patient, wherein an increase of the abundance of said species as detected in said sample compared to a sample from a non-colorectal adenoma and/or CRC patient is indicative for CRC for the species as listed in step b i), and indicative for colorectal adenomas for the species as listed in step b ii).

2. The method according to claim 1, wherein said CRC as diagnosed is localized CRC (stages 0, I, or II) and/or advanced colorectal adenomas.

3. The method according to claim 1 or 2, wherein said CRC is further distinguished from neoplasia-free (non-colorectal adenoma) in said non-CRC patient.

4. The method according to any one of claims 1 to 3, further comprising for CRC detecting an increase of the abundance of at least one of the species *Clostridium symbiosum, Clostridium hylemonae,* and *Lactobacillus salivarius,* and/or a decrease of the abundance of the species *Streptococcus salivarius,* and for colorectal adenomas detecting an increase of the abundance of at least one of the species *Clostridium saccharolyticum, Bacteroides fragilis, Clostridium bolteae, Holdermania filiformis, Ruminococcus lactaris, Clostridium perfringens, Oxalobacter formigenes, Bacteroides caccae,* and *Bacteroides coprocola* and/or a decrease of the abundance of at least one of the species *Streptococcus salivarius* and *Ruminococcus bromii.*

5. The method according to any one of claims 1 to 4, further comprising combining said test with a method selected from detecting the level of methylation in the gene wif-1, abundance or expression of KEGG module markers, abundance or expression of CAZy family markers, and Hemoccult FOBT test.

6. The method according to any one of claims 1 to 5, wherein said detecting comprises a method, such as a quantitative method, selected from the group of PCR, rtPCR, qPCR, multiplex PCR, high-throughput sequencing, metatranscriptomic sequencing, identification of strain-specific markers, such as genes and/or proteins, and 16S rDNA analysis.

7. A method for monitoring the progress of colorectal adenomas and/or colorectal cancer (CRC), comprising the steps of
a) obtaining a fecal sample comprising the gut microbiome from a human patient to be diagnosed,
b) detecting the fecal metagenome in said sample comprising the species
i) *Fusobacterium nucleatum* subsp. *vincentii, Fusobacterium nucleatum* subsp. *animalis, Poor-phyromonas asaccharolytica,* and *Peptostreptococcus stomatis,* and/or
ii) *Enterobacteriaceae bacterium* 9_2_54FAA, *Acidaminococcus intestini, Bacteroides stercoris,* and *Ruminococcaceae bacterium* D16, and
c) concluding on the progress of said colorectal adenomas and/or CRC in said patient, wherein an increase of the abundance of said species as detected in said sample compared to a sample from a non-progressing colorectal adenoma and/or CRC patient is indicative for progressive CRC for the species as listed in step b i), and indicative for progressing colorectal adenomas for the species as listed in step b ii).

8. The method according to claim 7, wherein said patient having said colorectal adenomas and/or colorectal cancer (CRC) has been diagnosed using a method according to any of claims 1 to 6.

9. The method according to claim 7 or 8, wherein said monitoring further comprises grading of said colorectal adenomas and/or said CRC into localized CRC (stages 0, I, or II) and/or advanced colorectal adenoma.

10. The method according to any one of claims 7 to 9, further comprising combining said test with a method selected from detecting the level of methylation in the gene wif-1, expression of KEGG module markers, expression of CAZy family markers, and Hemoccult FOBT test.

11. The method according to any of claims 1 to 10, wherein said patient is undergoing treatment for colorectal adenomas and/or CRC.

## Patentansprüche

1. Verfahren zur Diagnose von kolorektalen Adenomen und/oder kolorektalem Krebs (CRC), umfassend die Schritte von
a) Erhalten einer Fäkalprobe umfassend das Darmmikrobiom von einem menschlichen Patienten, der diagnostiziert werden soll,
b) Nachweisen des Fäkalmetagenoms in der Probe, umfassend die Spezies
i) *Fusobacterium nucleatum* subsp. *vincentii, Fusobacterium nucleatum* subsp. *animalis, Porphyromonas asaccharolytica,* and *Peptostreptococcus stomatis,* und/oder
ii) *Enterobacteriaceae bakterium* 9_2_54FAA, *Acidaminococcus intestini, Bacteroides stelcoris,* und *Ruminococcaceae bakterium* D16, und
c) Schlussfolgern auf kolorektale Adenome und/oder CRC in dem Patienten, wobei eine Zunahme der Häufigkeit der Spezies wie in der Probe nachgewiesen, verglichen mit einer Probe von einem nicht-kolorektalen Adenom und/oder CRC Patienten, für die Spezies wie in Schritt b i) aufgelistet CRC anzeigt, und für die Spezies wie in Schritt b ii) aufgelistet kolorektale Adenome anzeigt.

2. Verfahren nach Anspruch 1, wobei der CRC wie diagnostiziert lokalisierter CRC (Phasen 0, I oder II) und/oder fortgeschrittene kolorektale Adenome sind.

3. Verfahren nach Anspruch 1 oder 2, wobei der CRC weiter von Neoplasie-frei (nichtkolorektales Adenom) in dem nicht-CRC Patienten unterschieden wird.

4. Verfahren nach einem der Ansprüche 1 bis 3, weiter umfassend für CRC Nachweisen der Zunahme der Häufigkeit von mindestens einer der Spezies *Clostridium symbiosum, Clostridium hylemonae* und *Lactobacillus salivarius* und/oder einer Abnahme der Häufigkeit der Spezies *Streptococcus salivarius,* und für kolorektale Adenome Nachweisen der Zunahme der Häufigkeit von mindestens einer der Spezies *Clostridium saccharolyticum, Bacteroides fragilis, Clostridium bolteae, Holdermania filiformis, Ruminococcus lactaris, Clostridium perfringens, Oxalobacter formigenes, Bacteroides caccae* und *Bacteroides coprocola* und/oder eine Abnahme der Häufigkeit von mindestens einer der Spezies *Streptococcus salivarius* und *Ruminococcus bromii.*

5. Verfahren nach einem der Ansprüche 1 bis 4, weiter umfassend eine Kombination des Tests mit einem Verfahren ausgewählt aus Nachweisen des Spiegels an Methylierung in dem Gen wif-1, Häufigkeit oder Expression on KEGG Modul-Markern, Häufigkeit oder Expression von CAZy Familien-Markern und Hämokkult FOBT Test.

6. Verfahren nach einem der Ansprüche 1 bis 5, wobei der Nachweis ein Verfahren, wie zum Beispiel ein quantitatives Verfahren, ausgewählt aus der Gruppe von PCR, rtPCR, qPCR, Multiplex PCR, Hochdurchsatz-Sequenzierung, Metatranskriptom-Sequenzierung, Identifizierung von Stramm-spezifischen Markern, wie zum Beispiel Genen und/oder Proteinen und 16S rDNA Analyse umfasst.

7. Verfahren zur Überwachung des Fortschreitens von kolorektalen Adenomen und/oder kolorektalem Krebs (CRC), umfassend die Schritte von
a) Erhalten einer Fäkalprobe umfassend das Darmmikrobiom von einem menschlichen Patienten, der diagnostiziert werden soll,
b) Nachweisen des Fäkalmetagenoms in der Probe, umfassend die Spezies
i) *Fusobacterium nucleatum* subsp. *vincentii, Fusobacterium nucleatum* subsp. *animalis, Porphyromonas asaccharolytica* und *Peptostreptococcus stomatis,* und/oder
ii) *Enterobacteriaceae bacterium* 9_2_54FAA, *Acidaminococcus intestini, Bacteroides estercoris* und *Ruminococcaceae bacterium* D16, und
c) Schlussfolgern auf das Fortschreiten von kolorektalen Adenomen und/oder CRC in dem Patienten, wobei eine Zunahme der Häufigkeit der Spezies wie in der Probe nachgewiesen, verglichen mit einer Probe von einem nicht-progressiven kolorektalem Adenom und/oder CRC Patienten für die Spezies wie in Schritt b i) aufgelistet progressives CRC anzeigt, und für die Spezies wie in Schritt b ii) aufgelistet und für die Spezies wie in Schritt b ii) aufgelistet progressive kolorektale Adenome anzeigt.

8. Verfahren nach Anspruch 7, wobei der Patient, der die kolorektalen Adenome und/oder kolorektalen Krebs (CRC) aufweist, unter Verwendung des Verfahrens nach einem der Ansprüche 1 bis 6 diagnostiziert wurde.

9. Verfahren nach Anspruch 7 oder 8, wobei die Überwachung weiter eine Einteilung der kolorektalen Adenome und/oder CRC in lokalisiertes CRC (Phasen 0, I oder II) und/oder fortgeschrittenes kolorektales Adenom umfasst.

10. Verfahren nach einem der Ansprüche 7 bis 9, weiter umfassend eine Kombination des Tests mit einem Verfahren ausgewählt aus Nachweisen des Spiegels an Methylierung in dem Gen wif-1, Häufigkeit oder Expression on KEGG Modul-Markern, Häufigkeit oder Expression von CAZy Familien-Markern und Hämokkult FOBT Test.

11. Verfahren nach einem der Ansprüche 1 bis 10, wobei der Patient einer Behandlung gegen kolorektale Adenome und/oder CRC unterzogen wird.

## Revendications

1. Méthode de diagnostic d'adénomes colorectaux et/ou de cancer colorectal (CCR), comprenant les étapes consistant en:
a) l'obtention d'un échantillon fécal comprenant le microbiome intestinal d'un patient à diagnostiquer,
b) la détection du métagénome fécal dans ledit échantillon comprenant les espèces :
i) *Fusobacterium nucleatum* subsp. *vincentii, Fusobacterium nucleatum* subsp. *animalis,* Porphyromonas asaccharolytica, et Peptostreptococcus stomatis, et/ou ; ii) *Enterobacteriaceae bacterium* 9_2_54FAA, *Acidaminococcus intestini, Bacteroides stercoris,* et *Ruminococcaceae bacterium* D16, et
c) la conclusion de la présence d'adénomes colorectaux et/ou CCR chez ledit patient, méthode dans laquelle une augmentation d'abondance des dites espèces détectées dans ledit échantillon, comparé à un échantillon d'un patient sans adénome colorectaux et/ou sans CCR, est indicative de la présence de CCR sur la base des espèces indiquées en b i) et indicative d'adénomes colorectaux sur la base d'espèces indiquées en b ii)

2. La méthode selon la revendication 1, méthode dans laquelle le CCR diagnostiqué est un CCR localisé (stades 0, I, ou II) et/ou des adénomes colorectaux avancés.

3. La méthode selon la revendication 1 ou 2, méthode dans laquelle ledit CCR est divisé encore d'un sans neoplasie (non-adénome colorectal) dans ledit sans CCR.

4. La méthode selon chacune des revendications 1 à 3, méthode comprenant la détection d'une augmentation d'abondance d'au moins l'une des espèces *Clostridium symbiosum, Clostridium hylemonae,* et *Lactobacillus salivarius* et/ou une diminution d'abondance des espèces *Streptococcus salivarius,* et pour les adénomes colorectaux, une augmentation d'abondance d'au moins l'une des espèces *Clostridium saccharolyticum, Bacteroides fragilis*, *Clostridium boltege, Holdermania filiformis, Ruminococcus lactaris, Clostridium perfringens, Oxalobacter fromigens, Bacteroides caccae,* et *Bacteroides coprocola* et/ou une diminution en abondance d'au moins l'une des espèces *Streptococcus salivarius* et *Ruminococcus bromii.*

5. La méthode selon chacune des revendications 1 à 4, méthode laquelle comprend en plus la combinaison dudit test avec une méthode choisie dans le groupe de détection du niveau de méthylation d'un gène dit wif-1, détection d'abondance ou expression des marqueurs de module KEGG, détection d'abondance ou expression de marqueurs de la famille CAZy, et du test FOBT dit Hemoccult.

6. La méthode selon chacune des revendications 1 à 5, méthode de détection comprenant une méthode choisie dans le groupe comprenant, par exemple, une méthode quantitative, choisie dans le groupe comprenant la PCR, la rtPCR, la qPCR, la PCR multiplexe, le séquençage à haut débit, le séquençage métatranscriptomique, ou l'identification de marqueur spécifique de souche, tels que les gènes et/ou protéines, et analyse du gène codant pour l'ARNr 16S.

7. Méthode de surveillance la progression des adénomes colorectaux et/ou du cancer colorectal (CCR), méthode comprenant les étapes consistant en :
a) L'obtention d'un échantillon fécal qui comprend le microbiome intestinal d'un patient humain à diagnostiquer,
b) La détection du métagénome dans ledit échantillon comprenant les espèces : i) *Fusobacterium nucleatum* subsp. *vincentii, Fusobacterrium nucleatum* subsp. *animalis, Porphyromonas asaccharolytica,* et *Peptostreptococcus stomatis,* et/ou ii) *Enterobacteriaceae bacterium* 9_2_54FAA, *Acidaminococcus intestini, Bacteroides stercoris,* et *Ruminococcacege bacterium* D16, et
c) Faire une conclusion sur la progression desdites adénomes colorectaux et/ou CCR chez ledit patient, conclusion dans laquelle la constatation d'une augmentation d'abondance desdites espèces détectées dans ledit échantillon en comparaison d'un échantillon d'un patient sans adénome colorectal progressif ou sans CCR, est indicative de la présence de CCR progressif sur la base des espèces indiquées en b i) et indicative d'adénomes colorectaux progressifs sur la base d'espèces indiquées en b ii)

8. La méthode selon la revendication 7, méthode dans laquelle ledit patient atteint d'adénomes colorectaux et/ou cancer colorectal (CCR) étant diagnostiqué en utilisant une méthode selon chacune des revendications 1 à 6.

9. La méthode selon les revendications 7 ou 8, méthode de surveillance comprenant en plus la classification desdites adénomes colorectaux et/ou dudit CCR (stades 0, I, ou II) et/ou desdites adénomes colorectaux progressifs.

10. La méthode selon chacune des revendications 7 à 9, méthode comprenant en plus la combinaison dudit test avec une méthode choisie dans le groupe de la détection du niveau de méthylation d'un gène dit wif-1, de l'expression des marqueurs de module KEGG, de l'expression des marqueurs de la famille CAZy, et du test FOBT dit Hemoccult.

11. La méthode selon chacune des revendications 1 à 10, méthode dans laquelle le patient reçoit un traitement pour adénomes colorectaux et/ou CCR.
